# EUROPEAN PATENT SPECIFICATION

(11) **EP 0 702 683 B1**
(45) Date of publication and mention of the grant of the patent: **17.09.2003**
(21) Application number: 94916373.7
(22) Date of filing: 08.06.1994
(51) Int. Cl.: C07D 473/18

(54) **06-SUBSTITUTED GUANINE DERIVATIVES, A PROCESS FOR THEIR PREPARATION AND THEIR USE IN TREATING TUMOUR CELLS**
O6-SUBSTITUIERTE GUANINEDERIVATE, VERFAHREN ZU IHRE HERSTELLUNG UND IHRE ANWENDUNG FÜR BEHANDLUNG VON TUMORZELLEN
DERIVES DE GUANINE SUBSTITUEE EN POSTION 06, LEUR PROCEDE DE PREPARATION ET LEUR APPLICATION AU TRAITEMENT DES CELLULES TUMORALES

(30) Priority: 08.06.1993 IE 930432; 23.05.1994 GB 9410421
(43) Date of publication of application: 27.03.1996
(73) Proprietor: Cancer Research Technology Limited, London NW1 4JL (GB)
(72) Inventor: McMURRY, Thomas Brian Hamilton, Dublin 2 (IE); McELHINNEY, Robert Stanley, Dublin 2 (IE); McCORMICK, Joan Elizabeth, Dublin 2 (IE); ELDER, Rhoderick, Hugh c/o Christie Hospital, Wilmslow Road, Manchester M20 9BX (GB); Kelly, Jane Christie Hospital, Wilmslow Road, Manchester M20 9BX (GB)
(74) Representative: Parkes, Andrew John Aykroyd
(86) International application number: IE9400031
(87) International publication number: WO94029312

(56) References cited:
- WO-A-91/13898
- DE-A- 2 139 107
- J. MED. CHEM. vol. 37, no. 3 , 4 February 1994 pages 342 - 347 M-Y CHAE ET AL 'Substituted O6-Benzylguanine derivatives and their inactivation of human O6-alkylguanine-DNA transferase' cited in the application
- J. MED. CHEM. vol. 20, no. 3 , 1977 pages 341 - 344 A.P.MARTINEZ AT AL 'Potential Antitumour agents'
- J. MED. CHEM. vol. 18, no. 10 , 1975 pages 968 - 973 B. PAUL ET AL 'Inhibiotrs of nucleoside transport'

## Description

### Technical Field

The present invention relates to 0⁶-substituted guanine derivatives, a process for their preparation and their use in treating tumour cells. In particular, it relates to guanine derivatives having hetarylalkyl or naphthylalkyl substituents in the 0⁶ position, these compounds exhibiting the ability to deplete 0⁶-alkylguanine-DNA alkyltransferase (ATase) activity in tumour cells.

### Background Art

It has been suggested to use 0⁶-alkyl guanine derivatives possessing 0⁶-alkylguanine-DNA alkyltransferase depleting activity in order to enhance the effectiveness of chemotherapeutic alkylating agents used for killing tumour cells. There is increasing evidence that in mammalian cells the toxic and mutagenic effects of alkylating agents are to a large extent a consequence of alkylation at the 0⁶-position of guanine in DNA. The repair of 0⁶-alkylguanine is mediated by ATase, a repair protein that acts on the 0⁶-alkylated guanine residues by stoichiometric transfer of the alkyl group to a cysteine residue at the active site of the repair protein in an autoinactivating process. The importance of ATase in protecting cells against the biological effects of alkylating agents has been most clearly demonstrated by the transfer and expression of cloned ATase genes or cDNAs into ATase deficient cells: this confers resistance to a variety of agents, principally those that methylate or chloroethylate DNA. Whilst the mechanism of cell killing by 0⁶-methylguanine in ATase deficient cells is not yet clear, killing by 0⁶-chloroethylguanine occurs through DNA interstrand crosslink formation to a cytosine residue on the opposite strand via a cyclic ethanoguanine intermediate, a process that is prevented by ATase-mediated chloroethyl group removal or complex formation.

The use of 0⁶-methylguanine and 0⁶-n-butylguanine for depleting ATase activity has been investigated (Dolan et al., Cancer Res., (1986) **46**, pp. 4500; Dolan et al., Cancer Chemother. Pharmacol., (1989) **25**, pp 103. 0⁶-benzylguanine derivatives have been proposed for depleting ATase activity in order to render ATase expressing cells more susceptible to the cytotoxic effects of chloroethylating agents (Moschel et al., J. Med.Chem., 1992, **35** , 4486). U.S. Patent 5 091 430 and International Patent Application No. WO 91/13898 Moschel et al. disclose a method for depleting levels of 0⁶-alkylguanine-DNA alkyl-transferase in tumour cells in a host which comprises administering to the host an effective amount of a composition containing 0⁶-benzylated guanine derivatives of the following formula: 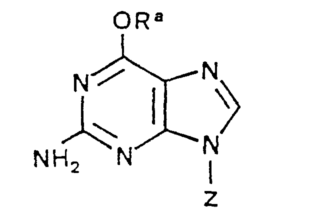 wherein Z is hydrogen, or 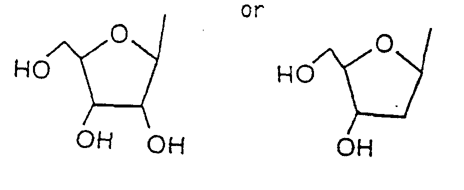 and R^{a} is a benzyl group or a substituted benzyl group. A benzyl group may be substituted at the ortho, meta or para position with a substituent group such as halogen, nitro, aryl such as phenyl or substituted phenyl, alkyl of 1-4 carbon atoms, alkoxy of 1-4 carbon atoms, alkenyl of up to 4 carbon atoms, alkynyl of up to 4 carbon atoms, amino, monoalkylamino, dialkylamino, trifluoromethyl, hydroxy, hydroxymethyl, and SOₙR^{b} wherein n is 0, 1, 2 or 3 and R^{b} is hydrogen, alkyl of 1-4 carbon atoms or aryl. Mi-Young Chae et al., J.Med.Chem., 1994, **37**, 342-347 - published after the priority date of the present application - describes tests on 0⁶-benzylguanine analogs bearing increasingly bulky substituent groups on the benzene ring or at position 9. Compound No. 6 described therein is 0⁶-(2-pyridylmethyl)guanine, which in this application is called 0⁶-(2-picolyl) guanine. However in the Results and Discussion at pages 342 - 343 of the Chae et al. paper, Compound No. 6 is not highlighted as being of interest but is grouped among "remaining compounds" which "exhibited intermediate activity" (Page 343 Lines 12 - 15 of the text). The authors confirm their earlier observations (J.Med.Chem., 1992, **35** 4486) that only allyl or benzyl substituents at the 0⁶ position of guanine efficiently inactivated ATase (page 343 lines 21 - 23 of the text).

0⁶-benzylguanine has limitations in its use as an ATase inactivator. It is more stable than would be desirable, resulting in a long survival time in an animal to which it is administered. It has a level of potential toxicity both alone and in combination with chloroethylating agents which is also undesirable and which may be related to the survival time.

The compounds of the present application exhibit different ATase inactivating characteristics from 0⁶-benzylguanine and in some cases the activity is up to 8 times greater than that of 0⁶-benzylguanine. Different half-life and toxicity characteristics have also been observed. Therefore, it is an object of the present invention to provide novel compounds useful for depleting ATase activity in order to enhance the effects of chemotherapeutic agents such as chloroethylating or methylating anti-tumour agents.

Another object of the invention is to provide pharmaceutical compositions containing compounds which are useful for depleting ATase activity. A further object of the present invention is to provide a method for depleting ATase activity in tumour cells. A still further object of the invention is to provide a method for treating tumour cells in a host.

### Disclosure of Invention

Accordingly, the present invention provides O⁶-substituted guanine derivatives of formula I: 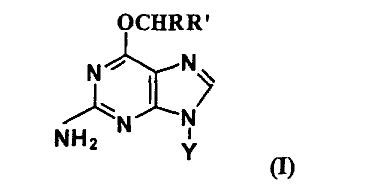 wherein
Y is H, ribosyl, deoxyribosyl, or R"XCHR''', wherein X is O or S, R" and R''' are C₁₋C₂₀alkyl, or substituted derivatives thereof having substitution by one or more of hydroxy, C₁-C₂₀alkoxy, amino, C₁-C₂₀alkylamino, amido or ureido;
R' is H or C₁-C₂₀alkyl or hydroxyC₁-C₂₀alkyl;
R is
(i) a cyclic group having at least one 5- or 6-membered heterocyclic ring, optionally with a carbocyclic or heterocyclic ring fused thereto, the or each heterocyclic ring having at least one hetero atom chosen from O, N, or S, or a substituted derivative thereof having substitution in the heterocyclic ring(s) and/or carbocyclic ring(s) by one or more of C₁-C₂₀alkyl, C₁-C₂₀ alkenyl, C₁-C₂₀alkynyl, halo, haloC₁-C₂₀alkyl, nitro, cyano, hydroxyC₁-C₂₀alkyl, SOₙR""where R"" is C₁-C₂₀alkyl and n = 0, 1 or 2, or a carboxyl or ester group of the formula - COOR⁵ wherein R⁵ is H or C₁-C₂₀alkyl; or
(ii) naphthyl or a substituted derivative thereof having substitution by one or more of C₁-C₂₀alkyl, C₁-C₂₀alkenyl, C₁-C₂₀alkynyl, halo, haloC₁-C₂₀alkyl, nitro, cyano, hydroxyC₁-C₂₀alkyl, SOₙR"" where R"" is C₁-C₂₀ alkyl and n = 0, 1 or 2, or a carboxyl or ester group of the formula - COOR⁵ wherein R⁵ is H or C₁-C₂₀alkyl;
and pharmaceutically acceptable salts thereof.

The present invention further provides 0⁶-alkylguanine derivatives of the formula 1: 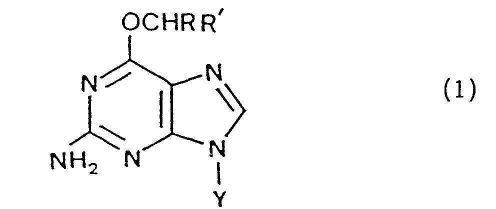 wherein
Y is H, ribosyl, deoxyribosyl, or R''XCHR''',
wherein X is 0 or S, R'' and R''' are C₁-C₂₀alkyl, or substituted derivatives thereof having substitution by one or more of hydroxy, C₁-C₂₀alkoxy, amino, C₁-C₂₀alkylamino, amido or ureido;
R' is H or C₁-C₂₀alkyl or hydroxyC₁-C₂₀alkyl;
R is
(i) a cyclic group having at least one 5 or 6 membered heterocyclic ring, optionally with a carbocyclic or heterocyclic ring fused thereto, the or each heterocyclic ring having at least one hetero atom chosen from 0, N, or S, or a substituted derivative thereof having substitution in the heterocyclic ring(s) and/or carbocyclic ring(s) by one or more of C₁-C₂₀alkyl, C₁-C₂₀alkenyl, C₁-C₂₀alkynyl, halo, haloC₁-C₂₀alkyl, nitro, cyano, hydroxyC₁-C₂₀alkyl, SOₙR'''' where R'''' is C₁-C₂₀alkyl and n = 0, 1 or 2.
(ii) naphthyl or a substituted derivative thereof having subsitution by one or more of C₁-C₂₀alkyl, C₁-C₂₀alkenyl, C₁-C₂₀alkynyl, halo, haloC₁-C₂₀alkyl, nitro, cyano, hydroxyC₁-C₂₀alkyl, SOₙR'''' where R'''' is C₁-C₂₀ alkyl and n = 0, 1 or 2.
and pharmaceutically acceptable salts thereof.

R may suitably be a 5- or 6-membered heterocyclic ring or a benzo derivative thereof, in which latter case the 0⁶-alkyl guanine moiety may be attached to R at either the heterocyclic or the benzene ring.

In preferred embodiments, R is a 5-membered ring containing S or O, with or without a second ring fused thereto.

Preferably, R is a heterocyclic ring having at least one S atom; more preferably, R is a 5-membered heterocyclic ring having at least one S atom; and most preferably, R is a thiophene ring or a substituted derivative thereof.

Alternatively, R may be a heterocyclic ring having at least one O atom, particularly, a 5-membered heterocyclic ring having at least one O atom and more particularly R may be a furan ring or a substituted derivative thereof.

As another alternative, R may be a heterocyclic ring having at least one N atom, particularly R may be a 6-membered heterocyclic ring having at least one N atom and in particular, R may be a pyridine ring.

In the definition of R, halo, haloalkyl, cyano, SOₙR"" where R"" is alkyl and n= 0, 1 or 2, and an ester group of the formula -COOR⁵ wherein R⁵is alkyl are preferred substituents.

An alkyl, alkenyl, or alkynyl group preferably contains from 1 to 10 and most preferably from 1 to 5 carbon atoms. Halo includes iodo, bromo, chloro or fluoro.

Examples of compounds of the invention (together with Compounds B.4214 and B.4218 not covered by the present application) are shown in Table 1.

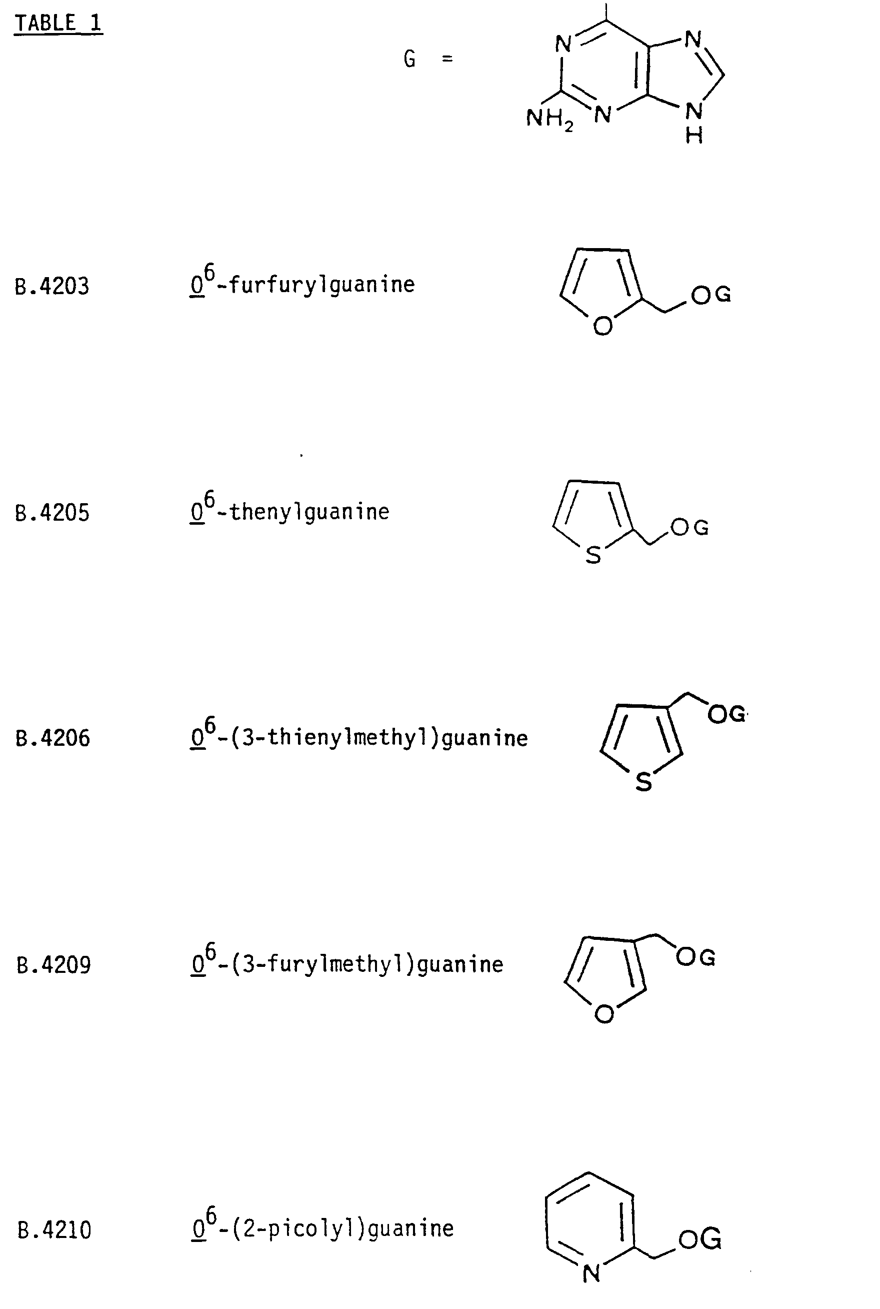

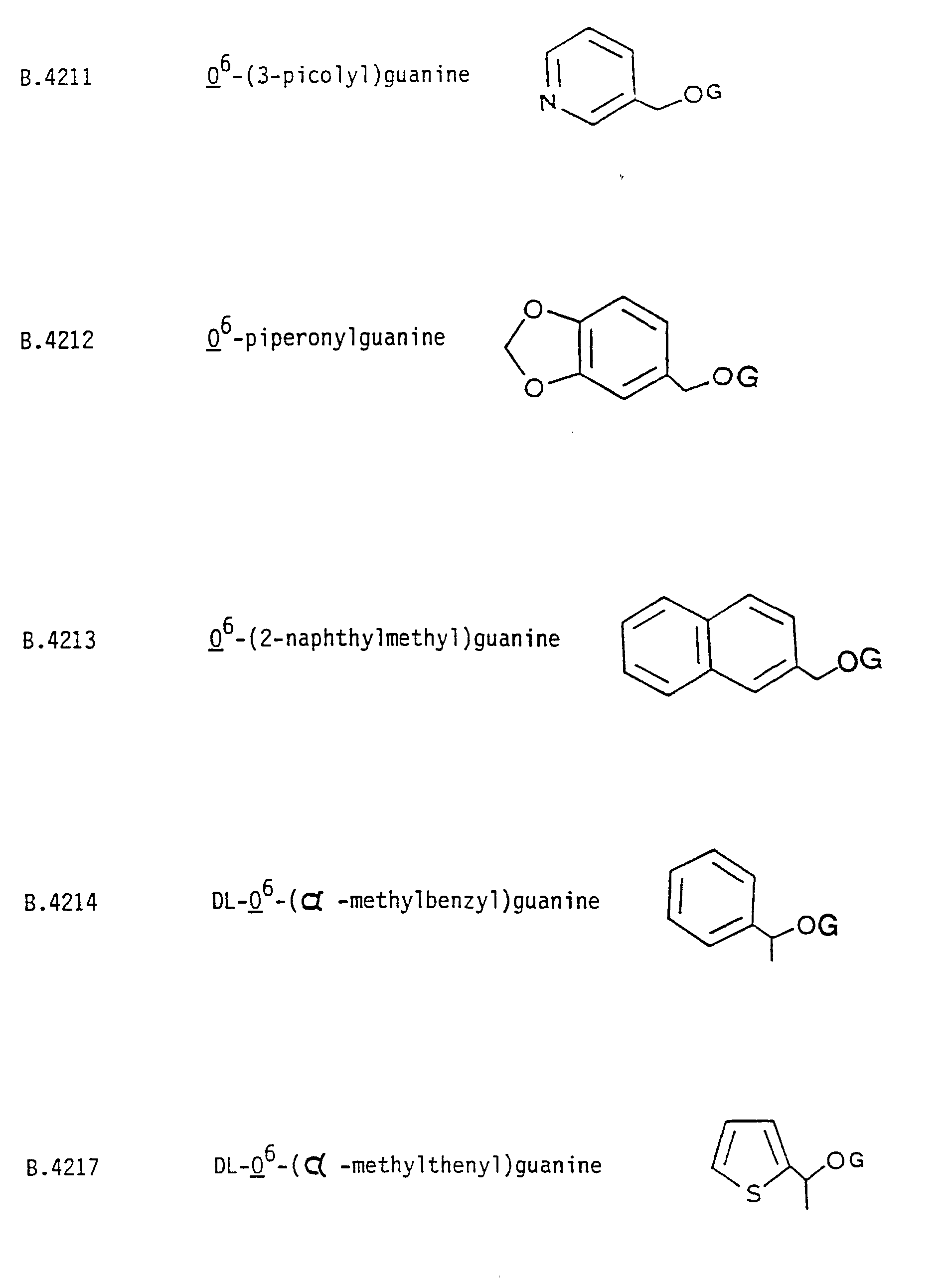

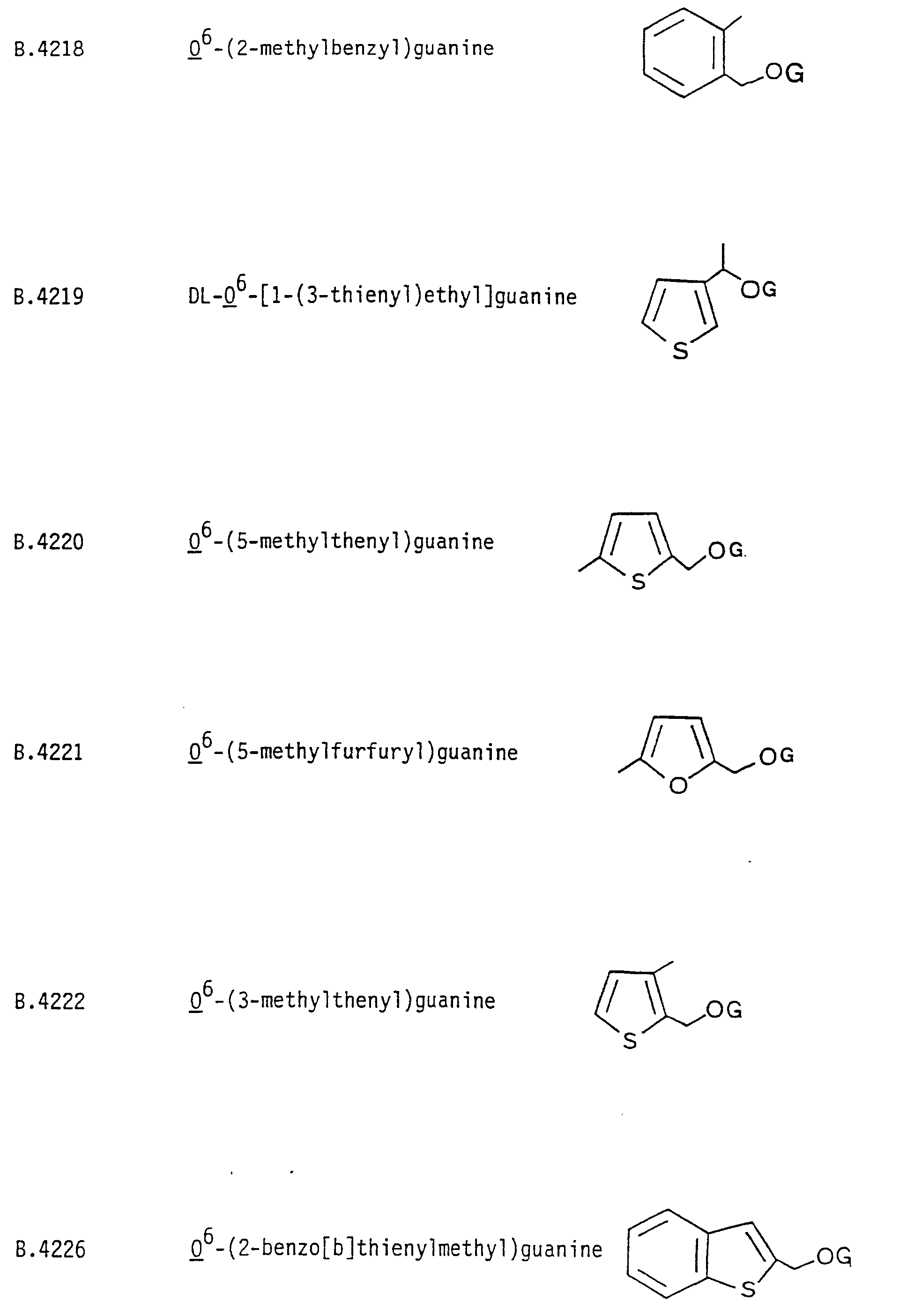

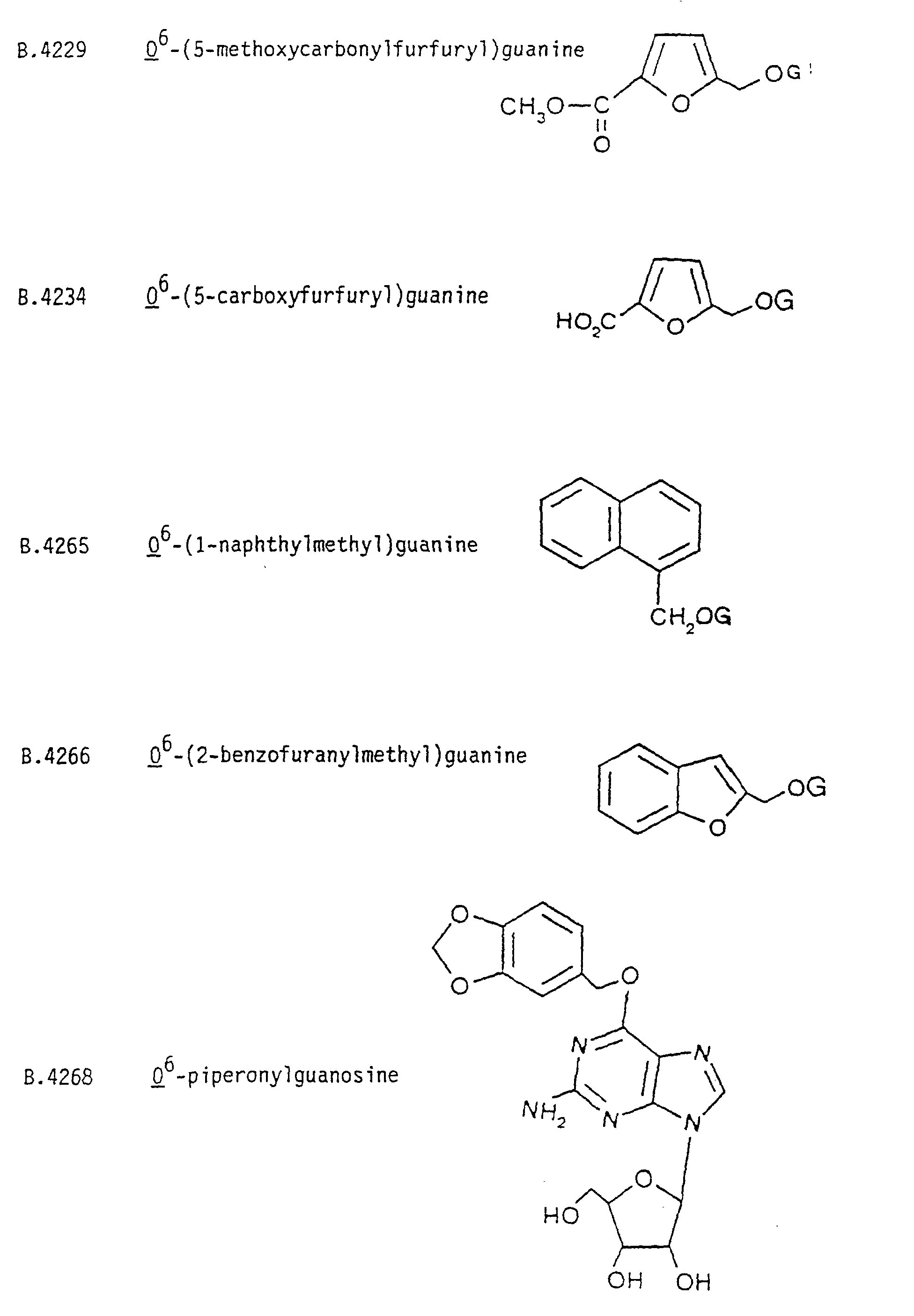

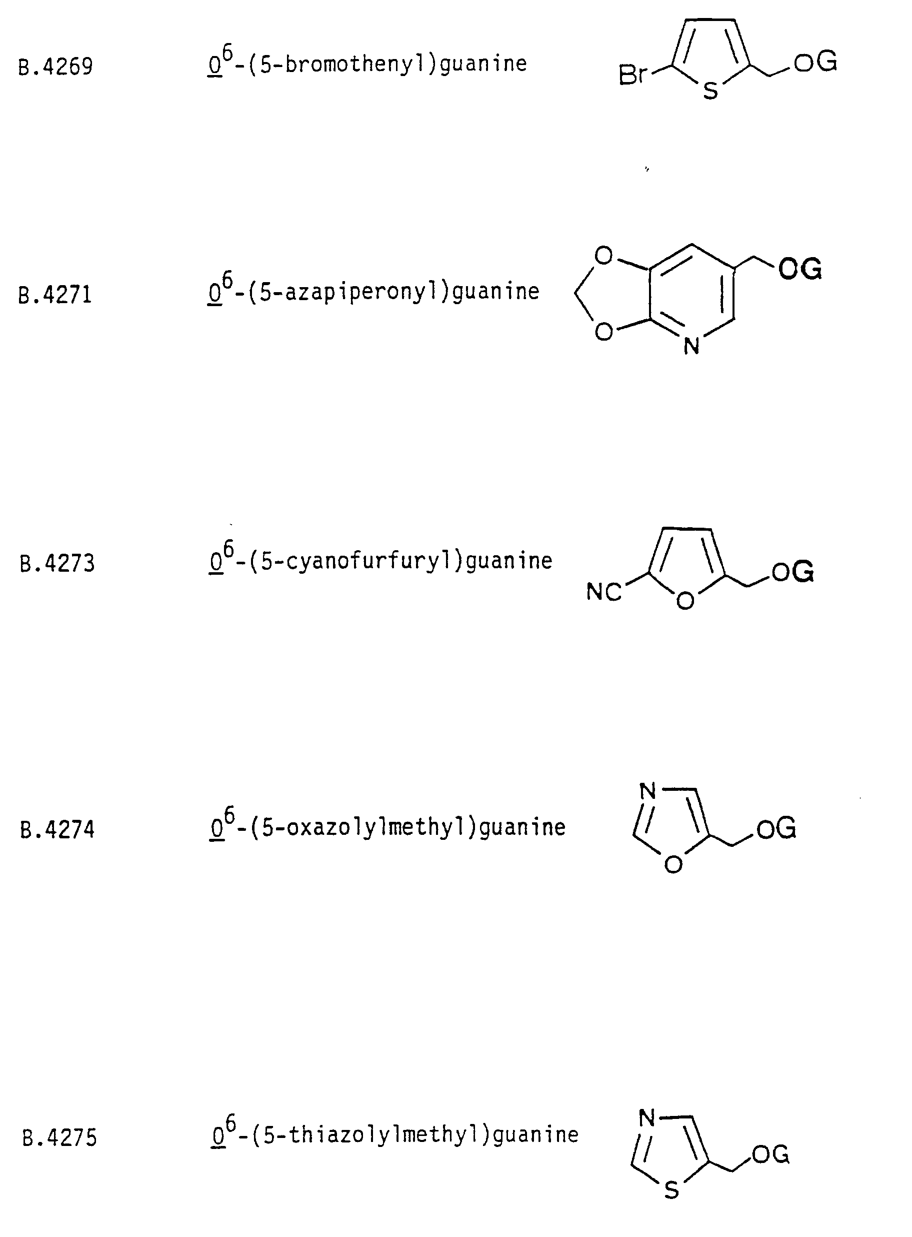

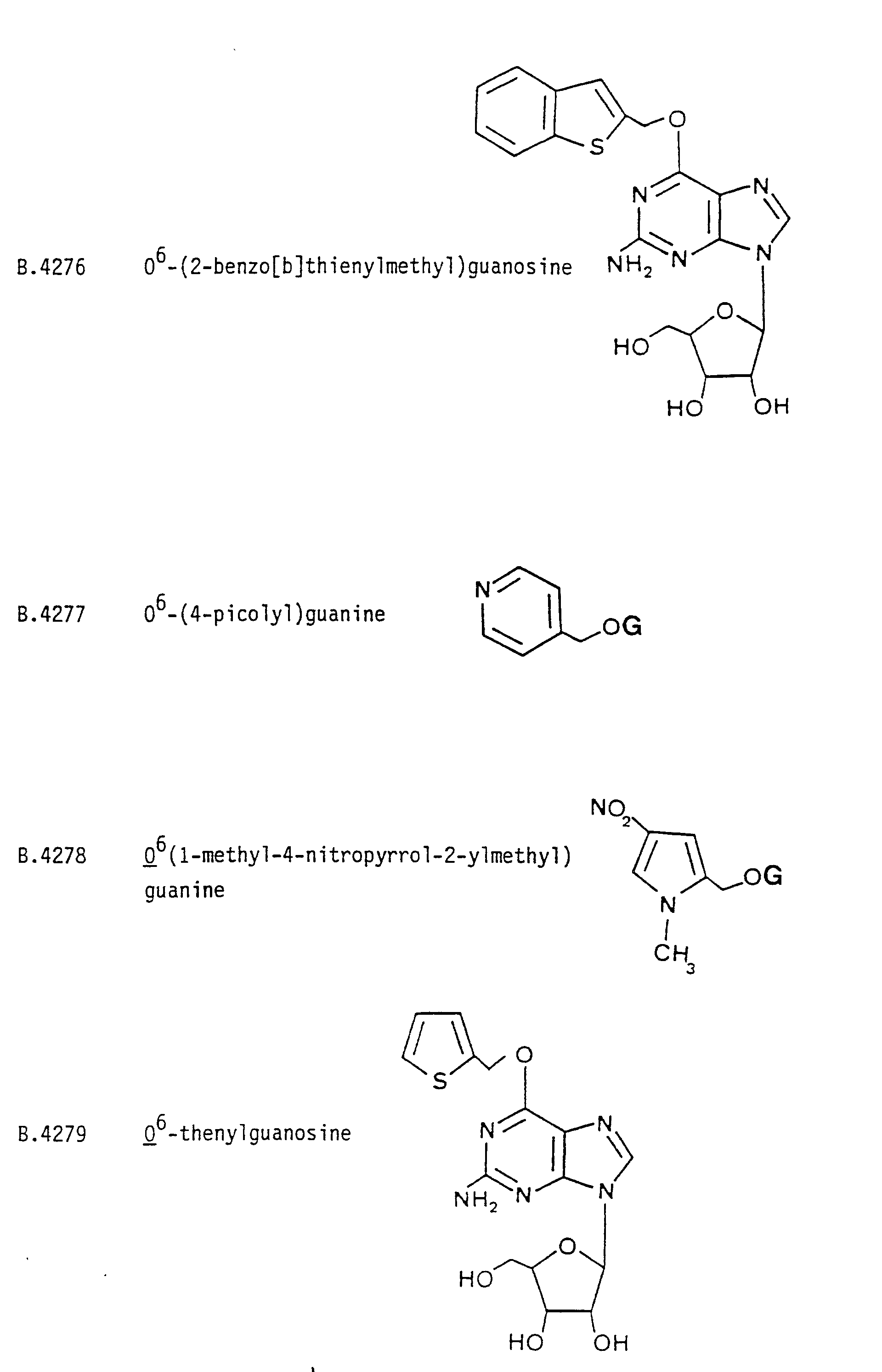

Among the compounds in Table 1 compounds B.4214 and B.4218 are not compounds of the invention. Compound B.4210 (ie the compound of Formula I in which R is 2-pyridyl, R' is H and Y is H) is not a preferred compound of the invention.

Particularly preferred compounds of the invention include:
B.4205 0⁶-thenylguanine
B.4206 0⁶-(3-thienylmethyl)guanine
B.4212 0⁶-piperonylguanine
B.4226 0⁶-(2-benzo[b]thienylmethyl)guanine
B.4266 0⁶-(2-benzofuranylmethyl)guanine
B.4275 0⁶-(5-thiazolylmethyl)guanine
and compounds substituted in the heterocyclic ring of R by a halo, cyano or ester group, including
B.4229 0⁶-(5-methoxycarbonylfurfuryl)guanine
B.4269 0⁶-(5-bromothenyl)guanine
B.4273 0⁶-(5-cyanofurfuryl)guanine.

Other preferred compounds include
B.4209 0⁶-3-furylmethylguanine
B.4276 0⁶-(2-benzo[b]thienylmethyl)guanosine
B.4277 0⁶(4-picolyl)guanine.

The most preferred compounds of the invention are those that inactivate ATase in vitro and/or in mammalian cells and/or tumour xenografts more effectively than O⁶-benzylguanine (BeG) and that sensitise mammalian cells and/or tumour xenografts to the killing or growth inhibitory effects of nitrosoureas and or methylating agents more effectively than BeG. Preferred compounds should also have, in comparison to BeG, reduced toxicity to normal tissues and/or to the entire organism when used in combination with such agents. Preferred compounds should not themselves be toxic or show more than minimal toxicity at the doses required to inactivate ATase, neither should any hydrolysis products of a preferred compound that was chemically unstable be toxic. Although the invention is not limited by any theory, preferred compounds may need to be less stable than BeG so that they undergo spontaneous chemical degradation soon after achieving maximal inactivation of ATase: in this way any action of metabolic processes that might act on the agent to generate toxic species would be minimised. Preferred compounds should be less able to sensitise human bone marrow or other normal cell types to the toxic effects of alkylating agents so that they would not exacerbate the known toxicity or generate new toxicities of these agents in normal human tissues.

Preferred compounds of the invention include those having a relatively low I₅₀ value in Table 4 herein (e.g. below 1.0µM, more particularly below 0.04µM) and/or having a relatively short half life in Buffer I (representing conditions for an in vitro assay) and/or Phosphate buffered saline (PBS) (representing conditions in physiological medium) in Table 4 herein (e.g. below 20 hours in Buffer I or below 16 hours in PBS).

A relatively short half-life can be regarded as an indicator that a compound of the invention would be less stable than 0⁶-benzylguanine due to the reactivity of RR'CH- and would tend to break down by hydrolysis in physiological medium.

The influence of the group RR'CH- in the compounds of formula I enabling them to act as ATase inhibitors is determined by electronic, steric and physicochemical factors. Steric factors may be related to the nature of the environment of the cysteine receptor site in ATase. Preferably R' is H. A secondary carbon atom attached to 0⁶ (as in the DL compounds B.4214 or B.4217) has been found to reduce greatly the inactivating activity, probably because of the bulk of the substituent.

Preferably the cyclic group in R does not have a methyl group in the vicinal position (as in B.4222) although the influence of vicinal substitution is evidently much less when R is heterocyclic than in the naphthyl isomers B.4213 and B.4265.

Physicochemical factors such as stability, solubility and water-lipid partition are relevant to the selection of compounds for use in vivo, affecting formulation, absorption and transport, for example. Selection of compounds may also be influenced by their differential distribution into different tissues.

One embodiment of the invention provides a pharmaceutical composition containing compounds of formula 1, wherein Y, R and R' are as defined above, or a pharmaceutically acceptable salt thereof, and a pharmaceutically acceptable excipient. Optionally the composition may also contain an alkylating agent such as a chloroethylating or methylating agent.

In a further embodiment, the present invention provides a method for depleting ATase activity in a host comprising administering to the host an effective amount of a composition containing a compound of formula 1 wherein Y, R and R' are as defined above, or a pharmaceutically acceptable salt thereof, more particularly a pharmaceutical composition as defined above. This method may alternatively be defined as a method of depleting ATase mediated DNA repair activity in a host.

The invention further provides a method for treating tumour cells in a host comprising administering to the host an effective amount of a composition containing a compound of formula I wherein Y, R and R' are as defined above or a pharmaceutically acceptable salt thereof, more particularly a pharmaceutical composition as defined above and administering to the host an effective amount of a composition containing an alkylating agent. The method may be used for treatment of neoplasms including those which are known to be sensitive to the action of alkylating agents e.g. melanoma and glioma and others whose resistance to treatment with alkylating agents alone may be overcome by the use of an inactivator according to the invention.

The invention also provides a process for preparing compounds of formula I comprising the steps of: reacting sodium hydride with a solution of RR'CHOH (wherein R and R' are as defined above) in an organic solvent, preferably at or below room temperature; adding 2-amino-N,N,N-trimethyl-1H-purin-6-aminium chloride or 2-amino-6-chloropurine riboside; treating the reaction mixture with weak acid and ether; and extracting the desired product.

### Brief Description of Drawings

The invention shall be described in greater detail with reference to the accompanying drawings in which:
Figure 1 is a graph of percentage residual activity of purified recombinant human ATase following incubation with different concentrations of various inactivators. Each point shows the mean of 3 measurements. The line at 50% residual activity is used for calculating I₅₀ values i.e. the concentration of inactivator required to produce a 50% reduction in ATase activity.
Figure 2 is two graphs of percentage cell growth against alkylating agent concentration (µg/ml), showing the sensitization effect of 0⁶-benzylguanine (BeG) and 0⁶-thenylguanine (B.4205) at two different concentrations (0.1 and 1.0µM) on sensitization of Raji cells to BCNU. The line at 90% growth is used for calculating D₉₀ values i.e. the dose of BCNU at which there was 90% growth as compared to untreated controls i.e. 10% growth inhibition.
Figure 3 is four graphs of percentages cell growth, against alkylating agent concentration (µM), showing the effect of BeG and B.4205 at four different concentrations (0.1, 0.5,1.0 and 5.0µM) on sensitization of Raji cells to temozolomide.
Figure 4 is a graph extrapolated from Figure 3 of values for D₅₀ (µM) (i.e. the dose of temozolomide at which there was 50% growth as compared to untreated controls) against inactivator concentration (µM).
Figure 5 is two graphs of percentage cell growth against inactivator concentration, showing the growth inhibiting effect of 0⁶-furfurylguanine (B.4203) and B.4205 on Chinese hamster V79 cells(RJKO) and a subline thereof (+120).
Figure 6 is two graphs of percentage cell growth against inactivator concentration, showing the growth inhibition effect of B.4203 and B.4205 on two Xeroderma pigmentosum subclones.
Figure 7 is four graphs of percentage cell growth against inactivator concentration (µM) showing the effect of degradation products of inactivators B.4203, B.4205, B.4212 (O⁶-piperonylguanine) and B.4226 (O⁶-[2-benzo(b)thienylmethyl)guanine) on Raji cell growth.
Figure 8 is a diagram of ATase activity (fm/mg) against time (hours) showing the depletion of ATase activity in A375 xenografts in nude mice for untreated controls, corn oil treated controls and BeG (30 mg/kg) and B.4205 (30 mg/kg) treated extracts.
Figures 9 - 13 are graphs of results of xenograft studies. In each figure the top graph shows percentage tumour volume against time (days) for A375 tumour xenografts in nude mice treated with BCNU alone, with BeG in combination with BCNU, and B.4205 in combination with BCNU. The lower graph in each figure shows the number of surviving mice in each treatment group against time (days) following the treatments illustrated in the top graph.

The particulars for each figure are as follows:-

| Figure | Mice | BCNU Concentration (mg/kg) | Inactivator (mg/kg) |
|---|---|---|---|
| 9 | ASU | Approx. 15 | 60 |
| 10 | ASU | 25 | 60 |
| 11 | OLAC | 10 | 30 |
| 12 | ASU | 16 | 60 |
| 13 | ASU | 16 | 60 |

Figure 14 is 3 graphs of percentage survival against temozolomide concentration (µM), showing the survival of bone marrow cells following treatment with inactivator (10µM) or DMSO (control) in combination with increasing doses of temozolomide.

### Modes for carrying out the Invention

0⁶-hetarylalkylguanine derivatives having ATase inactivating characteristics may be synthesized by adapting the standard preparation presented below as appropriate.

2-Amino-N,N,N-trimethyl-1H-purin-6-aminium chloride is prepared in accordance with the procedure described by Kiburis et al., J. Chem.Soc. (C), 1971, 3942. Details of the conditions for reaction of this quaternary salt with sodium benzyloxide (to give 0⁶-benzyl-guanine) not disclosed in MacCoss, Chen and Tolman, Tetrahedron Lett., 1985, **26**, 1815, were given in MacCoss, Tolman, Wagner and Hannah, European Patent Application No. 184,473, but these were not suitable for preparation of relatively sensitive analogues, and the standard preparation below was devised by the inventors.

### Standard preparation of 0⁶-hetarylalkylguanines (Formula I, y = H)

Sodium hydride (60% in oil; 0.8 g, 20 mmol) is added to a solution of RR'CHOH (56 mmol, ca. 5 ml) in DMSO (5 ml) and the mixture is stirred at room temperature for 1 hour. For solid or higher molecular weight alcohols, up to 10ml DMSO may be used instead of 5ml. 2-Amino-N,N,N-trimethyl-1H- purin-6-aminium chloride (2.29 g, 10 mmol) is added and stirring is continued for a further 1 hour. The change in UV spectrum is then complete (λ ₘₐₓ 312→ 284 nm) and the almost clear solution is treated with acetic acid (1.7 ml). After cooling and dilution with ether (300 ml), the mixture is set aside (2 hours) and the solid (A) collected. Trituration with water gives the product. A second fraction can be obtained by evaporation of the ether-DMSO filtrate and trituration of the residue successively with ether and water. Alternatively the product may be extracted from the solid (A) with warm acetonitrile. The recrystallised compounds show a single spot in TLC (C₆H₆-MeOH, 4:1) and are characterised by analysis and their NMR spectra. Frequently, they contain solvent of crystallisation. Melting points and analytical data are given in Table 2, UV and ¹H NMR data in Table 3. NMR spectra were measured on a Bruker WP80 or MSL 300 instruments.

This standard preparation procedure (with variations indicated by symbols in Table 2) was used to make the compounds listed in Tables 2a and 3a. In compounds B.4217 and B.4219, R' is methyl; in the remaining compounds R' is H. 0⁶-benzylguanine and Compounds B4214, B4218 and B4231 listed in Table 4 below were also made by this standard preparation procedure for comparative testing purposes.

The variations indicated by the symbols in Table 2 are as follows:
a 5 mmol sodium hydride per mmol quaternary salt are used in preparation of this compound. When the standard amount (2 mmol) is used, 40% of the quaternary salt can be recovered in the reaction work-up.
b This compound is made by hydrolysis of the methyl ester B.4229 (145 mg, 0.5 mmol) in 2-methoxyethanol (2.5 ml) and water (2.5 ml) by treatment with 2M-NaOH (2.5 ml) for 4 hours at room temperature. Neutralisation with acetic acid (0.32 ml, 5.5 mmol), gentle evaporation, trituration with water (3 ml) and filtration give a solid which on extraction with hot methanol yields the acid B.4234.
c The required figures are based on the monohydrate of a mixture of 4 parts of sodium salt of the acid and 3 parts of the acid, requiring Na, 4.3%. Found: Na, 4.44%.
d 3 mmol alcohol RCH₂OH per mmol quaternary salt are used instead of the standard 5.6 mmol.
e For alcohols which are too sensitive to sodium hydride in DMSO at room temperature, RCH₂ONa is prepared in DMF (2.5 ml at -10°C; 3 mmol RCH₂OH; 2 mmol sodium hydride). 1 mmol quaternary salt is added after 15-20 minutes and stirring continued for 2-3 hours at room temperature
f Products are extracted with acetonitrile.

### Preparation of Ribosides (formula I, Y= ribosyl)

A solution of alkoxide made as in the Standard Procedure above from sodium hydride (60% in oil; 120 mg, 3mmol) and RR'CHOH (4.6 mmol) in dry DMSO (2 ml) during 1 hour is treated with 2-amino-6-chloropurine riboside (302 mg, 1 mmol) and stirred for 5 minutes at room temperature, then 15 minutes at 60-65°C. The reaction is then complete as indicated by the change in UV spectrum (λₘₐₓ311→284nm). Cooling and thorough trituration with ether (100 + 15 ml) and filtration yield a solid which is treated with water (10 ml). The pH is brought from 11 to 8 by passing CO₂ briefly. Filtration removes inorganic material and the dried residue is extracted at room temperature with methanol (4 x 10 ml portions, each containing a drop of pyridine). Evaporation of almost all the methanol and addition of a little ether yield the product, almost pure by TLC (C₆H₆ -MeOH 4:1). It is recrystrallised from methanol and a trace of pyridine, dissolving and concentrating below 40°C, sometimes with final addition of a little ether.

This procedure was used to make the compounds listed in Tables 2b and 3b. Traces of impurities are difficult to remove but NMR spectra show that the nucleosides are ca. 90% pure. Yields are of the order of 30-40%.

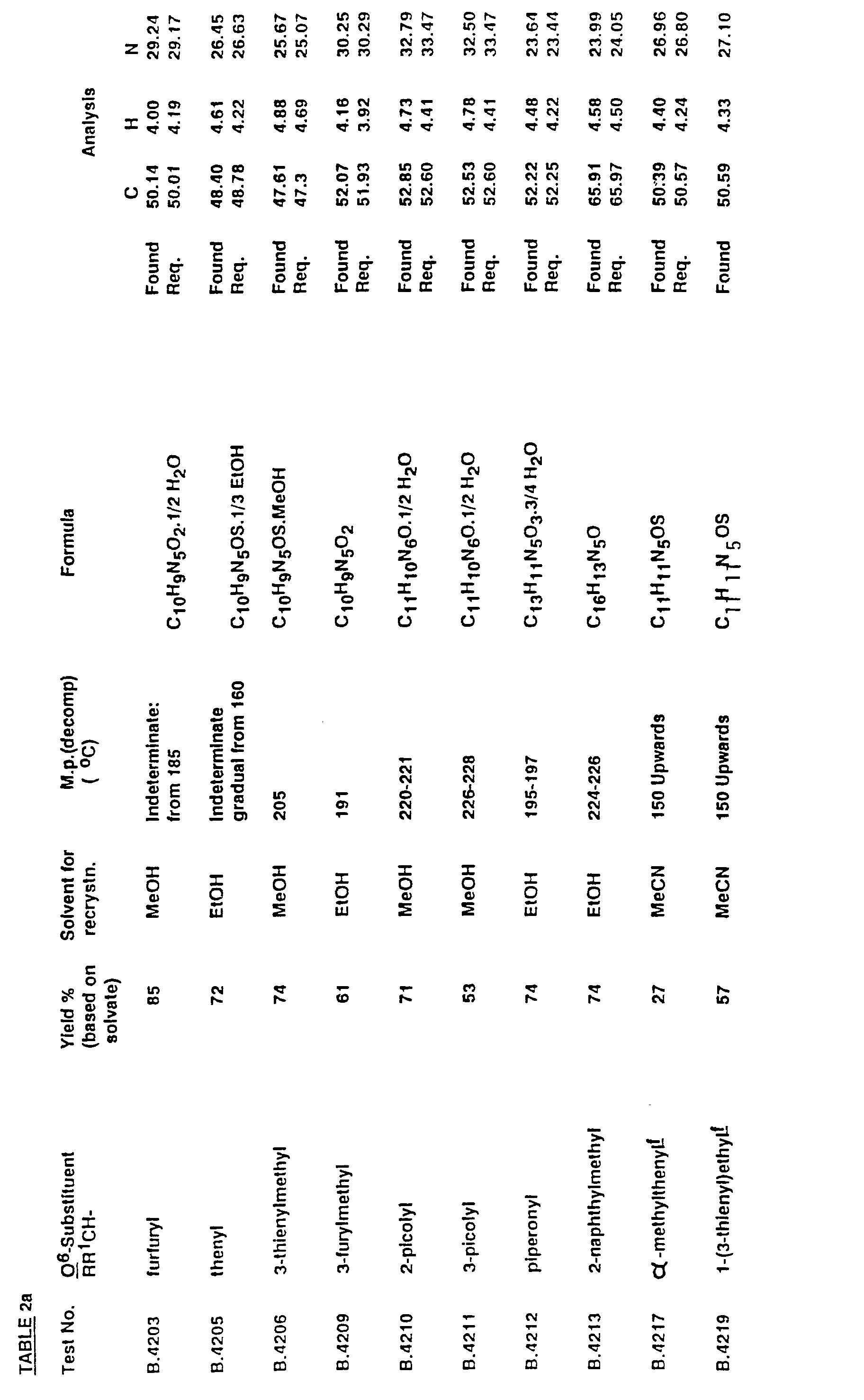

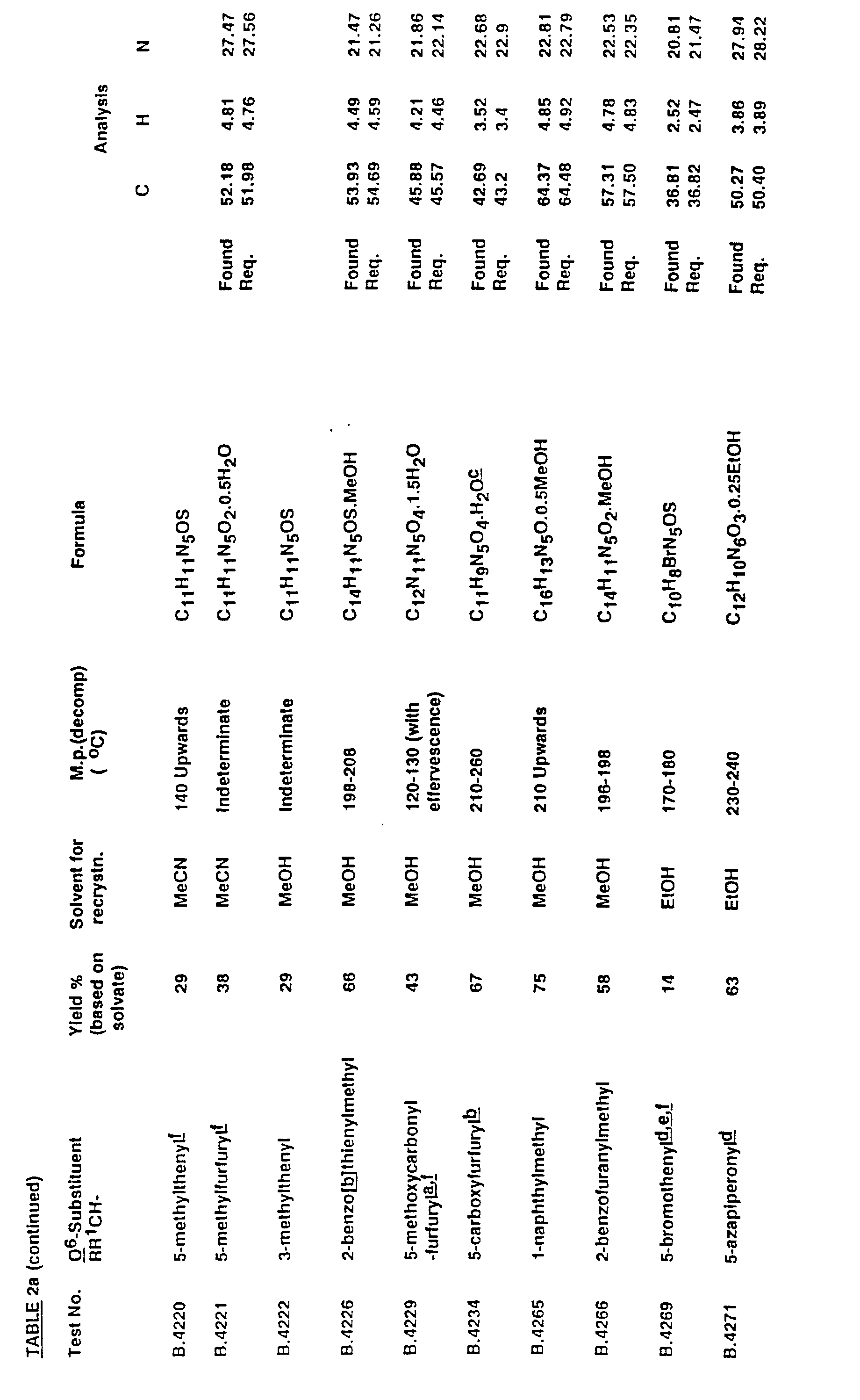

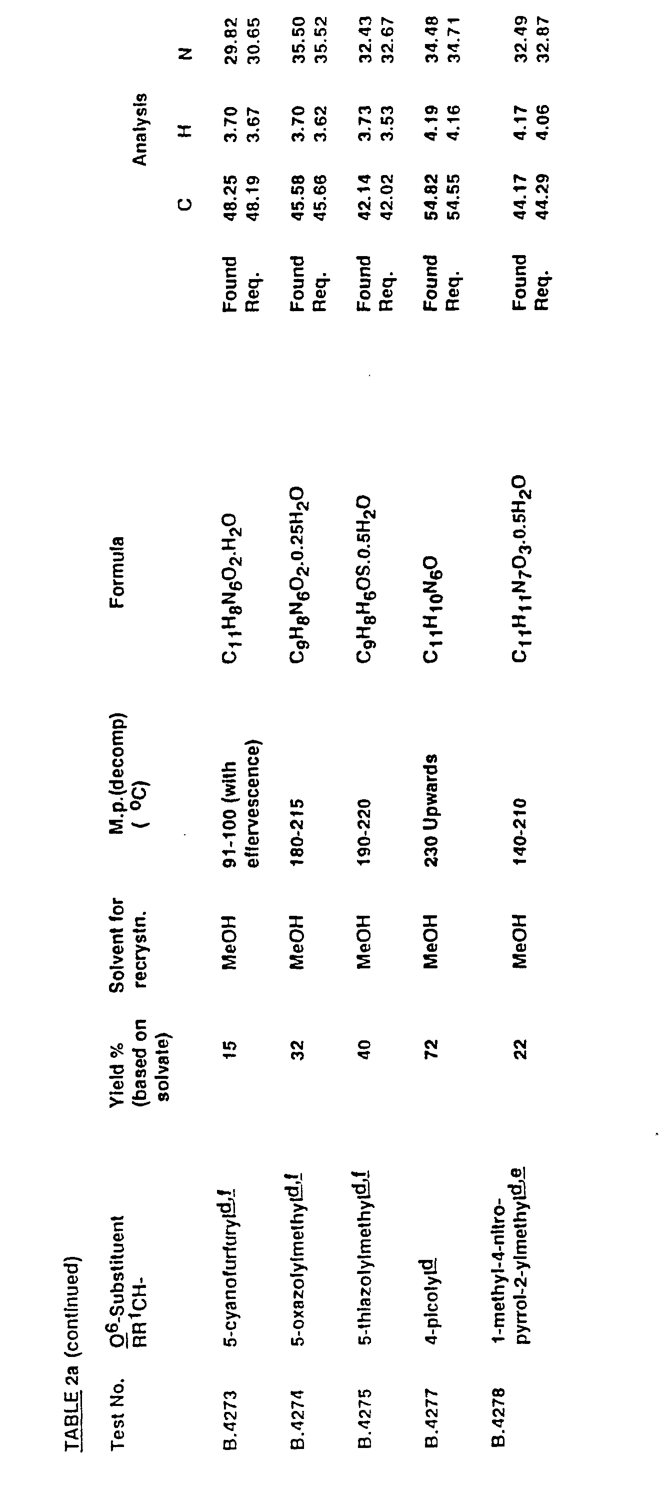

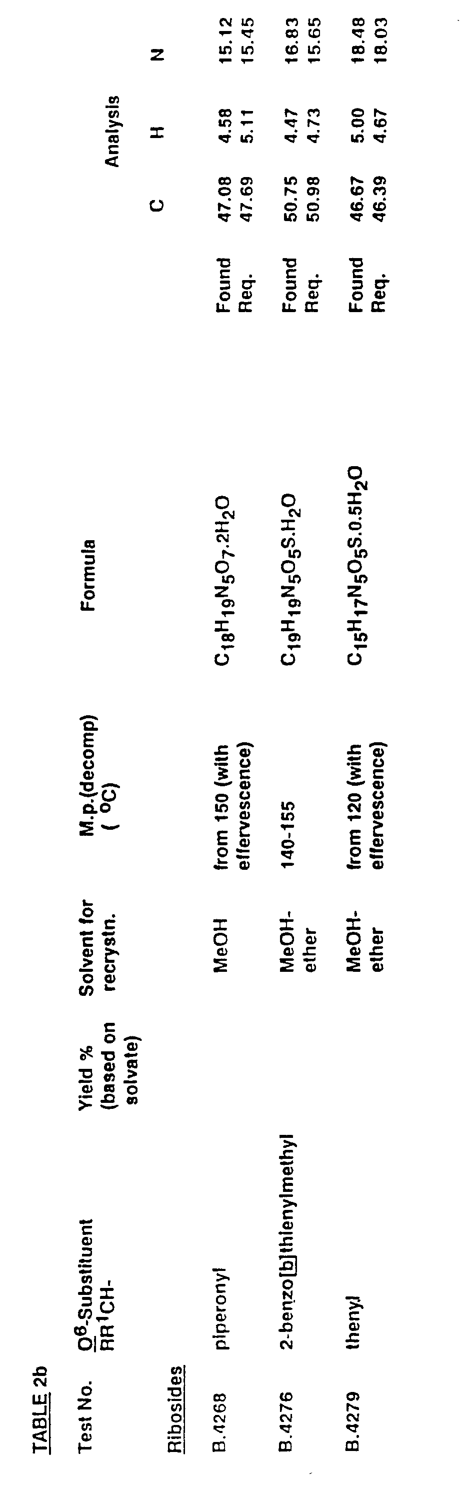

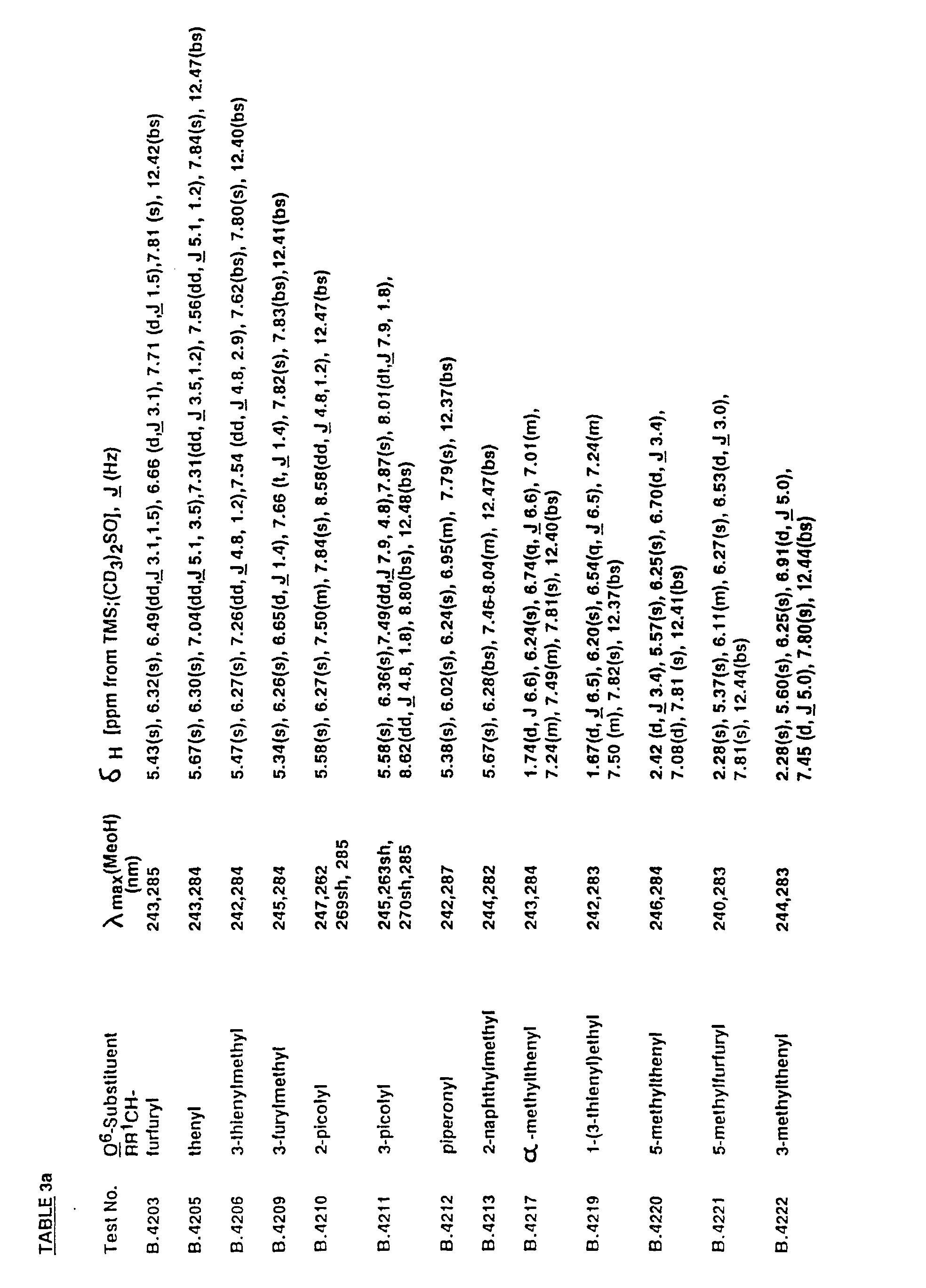

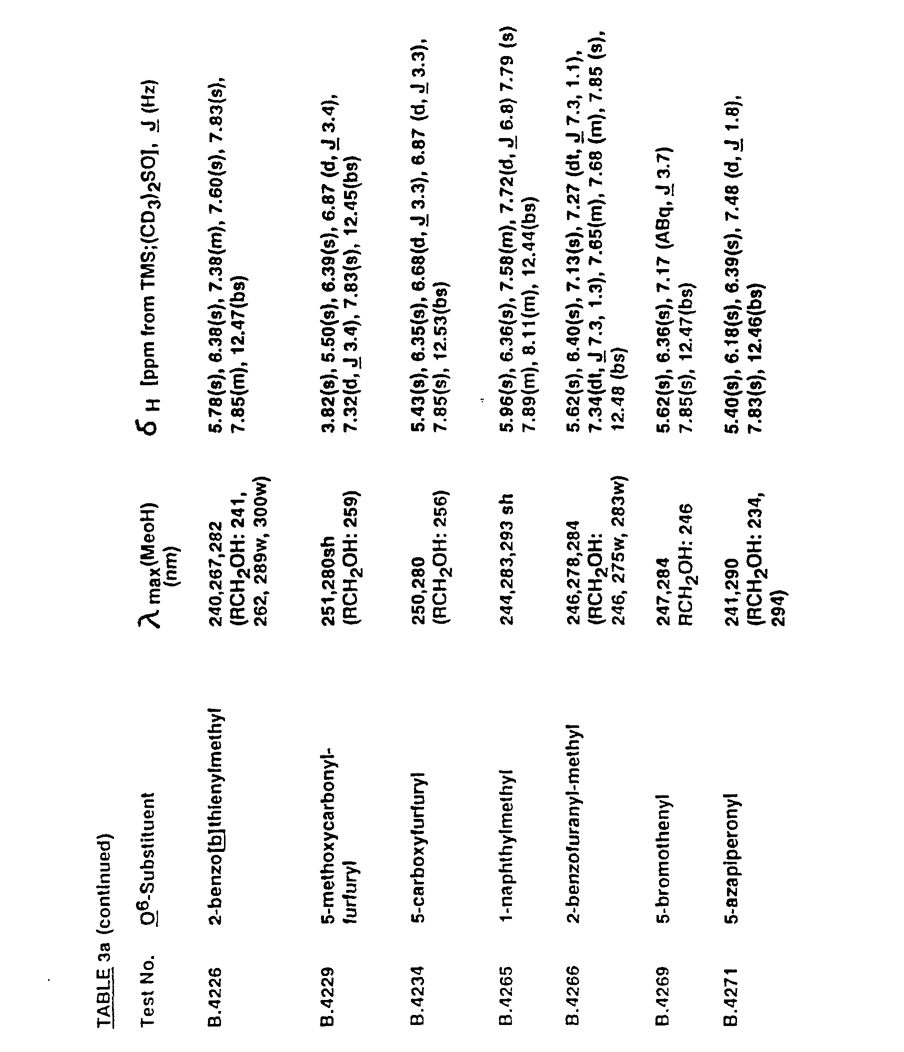

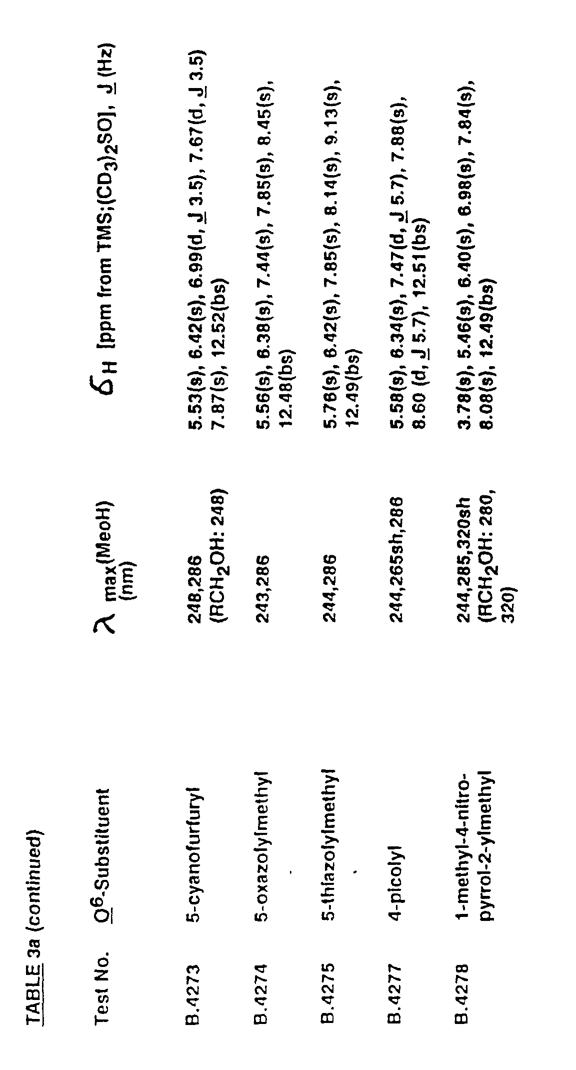

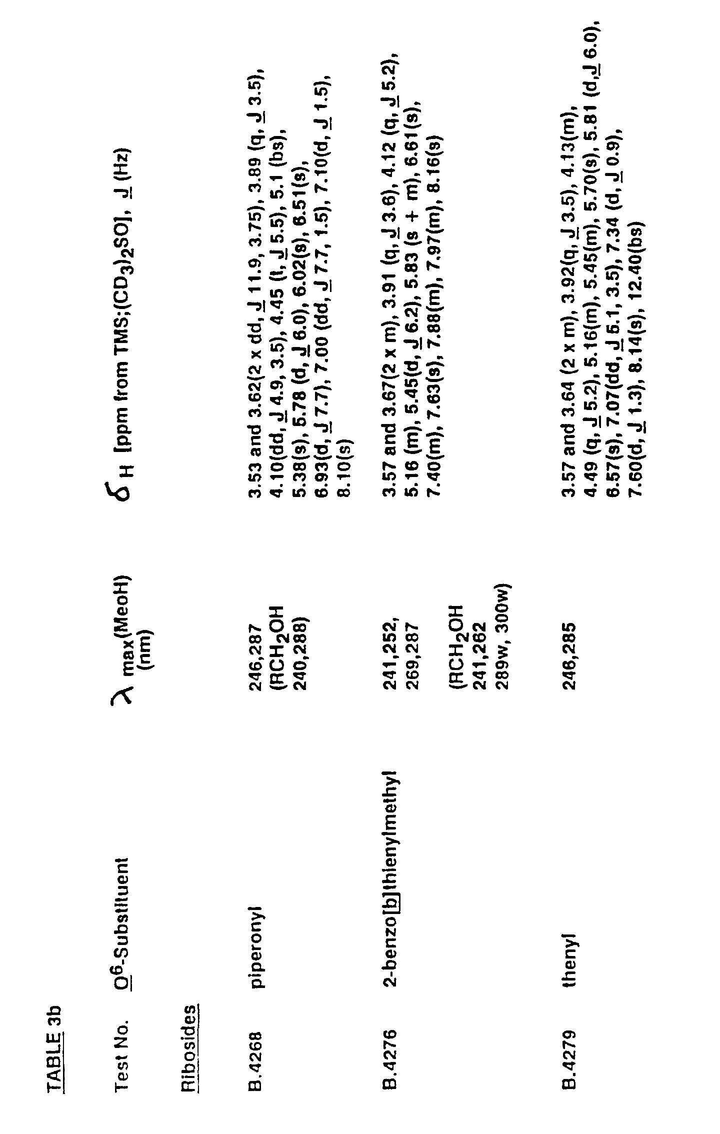

The starting alcohols RR'CHOH were usually made by reduction of the corresponding aldehydes, often commercially available, by sodium borohydride. A different approach was used for the precursors of the ester B.4229 and the nitrile B.4273. Sucrose was converted ¹ via 5-chloromethylfurfural into 5-hydroxymethylfurfural. Oxidation² of this aldehyde gave the carboxylic acid which was esterfied by the method of Bocchi et al.³ to methyl 5-(hydroxymethyl)furoate,⁴ required for B.4229. The oxime ⁵ of the aldehyde was dehydrated by the method of Carotti et al.⁶; the crude reaction product was treated with conc. aqueous ammonia in methanol before extraction into dichloromethane. Distillation afforded 5-cyanofurfuryl alcohol, ⁷ required for B.4273.

For B.4266, Vilsmeier reaction⁸ of benzofuran yielded the 2-aldehyde, reduced to the required alcohol,⁹ while for B.4226 lithiation and treatment with dimethylformamide gave the 2-aldehyde and in turn the alcohol.¹⁰

For B.4274, dimethyl tartrate was oxidised¹¹ to methyl glyoxylate which reacted ¹² with tosylmethyl isocyanide to give methyl oxazole-5-carboxylate.¹³ This was reduced by lithium aluminium hydride by the method of Fallab¹⁴ to the alcohol.¹⁵

For B.4275, bromomalonaldehyde¹⁶ and thiourea yielded 2-aminothiazole-5-carboxaldehyde.¹⁷ Deamination by amyl nitrite¹⁷ followed by sodium borohydride reduction gave 5-hydroxymethylthiazole¹⁴.

For B.4271, 5-azapiperonyl alcohol (m.p. 82-84°C; found, C, 54.60; H, 4.58; N, 9.09; C₇H₇NO₃ requires C, 54.90; H,4.61; N, 9.15%) was prepared from the corresponding aldehyde.¹⁸

For B.4278, 1-methylpyrrole-2-carboxaldehyde was nitrated¹⁹ and reduced to the alcohol ²⁰ by sodium borohydride.

By way of specific example, the preparation of 0⁶-thenylguanine (B.4205) will now be described:

### Preparation of 0⁶-thenylguanine

A solution of thenyl alcohol (3.18 ml, 33.6 mmol) in DMSO (3 ml) was treated with sodium hydride (60% in oil; 0.48g, 12 mmol), stirring cautiously at first. After 1 hour 2-amino-N, N, N-trimethyl-1H-purin-6-aminium chloride (1.37g, 6 mmol) was added and stirring continued 1 hour more. Acetic acid (1.0 ml) was added, cooling briefly, and the mixture diluted with ether (180 ml). The solid (2.09g) was collected after 1-2h. Evaporation of ether from the filtrate and distillation of DMSO and excess thenyl alcohol (b.p. 48-57°C/0.2 mm) left a residue which on trituration with ether yielded a second solid fraction (0.36g). The combined solids were rubbed (trituration) with water (6 ml), yielding product (1.335g, 90%) showing a strong spot in TLC (C₆H₆-MeOH, 4:1) with only traces of impurity. Dissolution in hot ethanol (30 ml), clarification by filtration through Celite, and concentration (to 10 ml) using a rotary evaporator yielded B.4205 (1.125g, 71% of material containing 1/3 EtOH per mole as solvate).

Compounds of formula I in which Y is R''XCHR''' (seco-nucleosides) may be prepared by an analogous preparation to the reaction of 0⁶-benzylguanine with α-chloro-ethers (MacCoss et al., Tetrahedron Lett.; European Patent Application No. 184,473., loc. cit.) or with alkyl bromides (e.g. Kjellberg, Liljenberg and Johansson, Tetrahedron Lett., 1986, **27**, 877; Moschel, McDougall, Dolan, Stine, and Pegg, J.Med. Chem., 1992, **35**, 4486).

Typical "sugar" components corresponding to R''XCHR''', leading to seco-nucleosides, are made by methods described in e.g. McCormick and McElhinney, J. Chem. Soc., Perkin Trans. 1, 1985, 93; Lucey, McCormick and McElhinney, J. Chem. Soc. Perkin Trans. 1, 1990, 795.

Compounds of formula I in which Y is ribosyl or deoxyribosyl (nucleosides) may be prepared by methods analogous to the syntheses of 0⁶-benzylguanine riboside and 2-deoxyriboside (Moschel et al. 1992; cf. Gao, Fathi, Gaffney et al., J. Org. Chem., 1992, **57**, 6954; Moschel, Hudgins and Dipple, J. Amer. Chem. Soc., 1981, **103**, 5489) (see preparation of Ribosides above).

### Industrial Applicability

The amount of the compound of the present invention to be used varies according to the effective amount required for treating tumour cells. A suitable dosage is that which will result in a concentration of the compound of the invention in the tumor cells to be treated which results in the depletion of ATase activity, e.g. about 1 - 2000 mg/kg, and preferably 1 - 800 mg/kg, particularly 1-120 mg/kg, prior to chemotherapy with an alkylating agent.

The pharmaceutical composition of the invention may be formulated in conventional forms with conventional excipients, as described for example in WO 91/13898, the contents of which are incorporated herein by reference. The composition may contain the inactivator according to the invention together with an alkylating agent; or the composition may comprise two parts, one containing the inactivator and the other containing the alkylating agent. The method of administering the compounds of the invention to a host may also be a conventional method, as described in WO 91/13898 for example. For administration of an inactivator according to the invention to patients, the pharmaceutical composition may suitably contain the inactivator in a suitable vehicle such as 40% polyethyleneglycol 400 in saline solution, or in saline or 3% ethanol (in saline), for intravenous injection, or in a powder form in suitable capsules for oral administration.

Alkylating agents may be administered in accordance with known techniques and in conventional forms of administration, as described in WO 91/13898 for example or preferably as a single dose immediately after or up to 24 hours after but preferably around 2 hours after administration of the ATase inactivating agents and also at doses lower than those used in standard treatment regimen. A reduction in dose may be necessary because the inactivators would generally be anticipated to increase the toxicity of the alkylating agents. Examples of chloroethylating agents include 1,3 bis (2-chloroethyl) -1-nitrosourea (BCNU), 1-(2-chloroethyl)-3-cyclohexyl-1-nitrosourea (CCNU), fotemustine, mitozolomide and clomesone and those described in McCormick, McElhinney, McMurry and Maxwell J. Chem. Soc. Perkin Trans. I, 1991, 877 and Bibby, Double, McCormick, McElhinney, Radacic, Pratesi and Dumont Anti-Cancer Drug Design, 1993, **8**, 115. Examples of methylating agents include temozolomide (British Patent GB 2 104 522) and dacarbazine, procarbazine, and streptozocin.

### METHODS

### Purification of Recombinant ATases

The cDNA cloning and overexpression of the human ATase has been reported previously²³. Purification of the recombinant proteins was achieved either by affinity chromatography through a DNA-cellulose column as described by Wilkinson et al.,^{25, 26}, or by DEAE-cellulose ion-exchange chromatography. For the latter, the ATase protein was partially purified by ammonium sulphate precipitation (30 - 60%) and dialysed against 10 mM Tris-HCl pH 7.5, 1 mM DTT, 2 mM EDTA, 10% glycerol, before loading on a DEAF-cellulose column. The ATase was then eluted with a 0-0.1 M NaCl gradient. The purified human ATase protein retained activity for more than one year when stored at high concentration at -20°C in buffer I [50 mM-Tris/HCl (pH 8.3)/3 mM-dithiothreitol/1 mM-EDTA] and could be thawed and refrozen several times without substantial loss of activity.

### Incubation with Inactivators and ATase Assay

Compounds to be tested were dissolved in DMSO to a final concentration of 10 mM and diluted just before use in buffer I containing 1 mg/ml bovine serum albumin (IBSA). Recombinant ATase was diluted in IBSA and titrated in order that the reaction be conducted under ATase, and not substrate, limiting conditions. In each assay, fixed amounts of ATase (60-75 fmol) were incubated with varying amounts of 0⁶-benzylguanine, or test compound in a total volume of 200 µl of IBSA containing 10 µg of calf thymus DNA at 37°C for 1 hour. The [³H]-methylated-DNA substrate (100 µl containing 6.7 µg of DNA and 100 fmol of 0⁶-methylguanine) was added and incubation continued at 37° for 1 hour, until the reaction was completed. Following acid hydrolysis of the DNA as previously described²¹ the [³H]-methylated protein was recovered and quantitated by liquid scintillation counting. Samples were typically assayed in duplicate and experiments repeated several times. I₅₀ is the concentration of inactivator required to produce a 50% reduction in ATase activity.

### Cell Culture and preparation of extracts

Mammalian cells were cultured under standard conditions. For example, Raji (a human lymphoblastoid cell line from a Burkitt's lymphoma) cells were grown in suspension culture in RPMI medium supplemented with 10% horse serum. A375M cells are human melanoma cells from which the xenografts described below were established following subcutaneous injection into nude mice: WiDr cells are a human colon carcinoma cell line: Hamster+120 cells are a mitozolomide-selected subline of a Chinese hamster lung fibroblast V79 cell line called RJKO: Yoshida cells are a rat carcinosarcoma cell line and YR_{bus} is a busulphan resistant subline thereof: XP cells are an SV40-transformed fibroblast cell line originally from the skin of a Xeroderma pigmentosum patient, pHMGhAT2b cells are a clone of these cells that have been transfected with a mammalian cell expression vector containing the human ATase cDNA and pHMGla cells are a clone that have been transfected with the expression vector only (i.e. one not containing the human ATase cDNA). Cell pellets were resuspended in cold (4°C) buffer I containing 2 µg/ml leupeptin and sonicated for 10 seconds at 12 µ peak to peak distance. After cooling in ice, the cells were sonicated for a further 10 seconds at 18 µ. Immediately after sonication, 0.01 volumes of 8.7 mg/ml phenylmethanesulphonylfluoride in ethanol was added and the sonicates centrifuged at 15 000 g for 10 minutes at 4°C to pellet cell debris. The supernatant was kept for determination of ATase activity (see below).

### Stability of Inactivators at 37°C.

Inactivators (10mM in DMSO) were diluted to 0.1mM in prewarmed degassed buffer I (1mM EDTA, 50mM Tris pH 8.3) or PBS (pH 7-7.2). PBS (Phosphate buffered saline) is 0.8% NaCl, 0.02% KCl, 0.15% Na₂H₂PO₄, 0.02% KH₂PO₄, pH 7.2. Samples were immediately transferred to a CARY13 spectrophotometer (cuvette block held at 37°C) and scanned at an appropriate wavelength (according to the spectral properties of the compound) at 3-10 minute intervals for up to 80 hours. The results were expressed as percentage absorbance change versus time and T1/2 values (half life) extrapolated from this.

### Inactivation of ATase activity in mammalian cells.

Cells were diluted to 10⁶/ml in culture medium containing either the appropriate concentration of inactivator or an equivalent volume of vehicle (DMSO). Following incubation at 37°C for 2 hours the cells were harvested by centrifugation, washed twice with PBS and the resulting cell pellets (between 1-2 x 10⁷ per pellet) stored at -20°C. ATase activity was determined as described above, in duplicate sonicated cell extracts and expressed as the percentage activity remaining based on that present in the untreated controls (for example 350-450 fm/mg in Raji cells). I₅₀ (i.e concentration of inactivator required to reduce ATase activity by 50%) values were extrapolated from this data.

### Sensitization of Raji and A375M cells to BCNU and temozolomide.

Sensitization of Raji cells to the cytotoxic effects of BCNU and temozolomide following a 2 hour pretreatment with inactivator was analysed using an XTT assay²². Briefly, cells were plated at 1000 cells/well in 96 well plates and incubated at 37°C for 30 minutes prior to the addition of medium containing either the appropriate concentration of inactivator or an equivalent volume of vehicle. Following a 2 hour incubation at 37°C, medium containing either increasing doses of BCNU, temozolomide or equivalent vehicle was added and the cells allowed to grow for 6 days. At this time XTT solution was added and the cells incubated for a further 4 hours at 37°C. The resulting red/orange formazan reaction product was quantified by measuring absorption at 450nm on a microtitre platereader.

Sensitization of A375M cells to the cytotoxic effects of BCNU was analysed by MTT assay ²⁴ differing from the XTT assay described above as follows. A375M cells (1000 per well) were allowed to grow for 24 hours then treated with the inactivator and 2 hours later with BCNU. After 6 days MTT solution (4mg/ml) was added and cells incubated for 3 hours at 37°C. Medium was aspirated and the resulting purple formazan crystals were solubilised in DMSO (120µl) and cell viability was quantified by measuring absorption at 540nm using a microtitre plate reader.

From this data the percentage growth of cells relative to that in control wells was determined for a range of BCNU or temozolomide doses in both the presence and absence of inactivator. Raji sensitization to BCNU (D₉₀.^{C}/D₉₀.^{I}) was determined by dividing the D₉₀ (i.e. dose at which there was 90% growth versus untreated controls i.e. 10% growth inhibition) calculated for data on use of BCNU alone (D₉₀.^{C}) by that for BCNU plus inactivator (D₉₀.^{I}). A value of one (1) thus indicates no sensitization by the inactivator. Raji sensitization to temozolomide and A375M sensitization to BCNU were determined using the corresponding D₅₀ values (i.e. the doses at which there was 50% growth inhibition).

### Sensitivity of mammalian cells to the inactivators or their hydrolysis products

In order to assess the effects on cell growth alone, Xeroderma pigmentosum cells and Chinese Hamster V79 cells were exposed to increasing concentrations (up to 600µM) of selected inactivators for 2h at 37°. In some cases, the inactivators were allowed to undergo hydrolysis at 37°C for 20 hours and then added to Raji cells in order to assess the extent to which the decomposition products of the agents might inhibit cell growth. After 6 days the extent of cell growth was determined as described above.

### Sensitization of bone marrow cells to temozolomide (GM-CFC assay)

For the granulocyte/macrophage colony-forming cell (GM-CFC) assay primary human bone marrow samples were obtained from patients undergoing cardiothoracic surgery. Following removal of erythrocytes from the samples, cells were plated out at 1-2 x 10⁵/ml in 300mOsM Iscoves medium containing 10µM inactivator or equivalent volume of DMSO, 20% foetal calf serum, 10% 5637 conditioned medium as a source of growth factors and 0.3% agar noble, in 1ml petri dishes containing appropriate dose of temozolomide and incubated at 37°C in an atmosphere of 5% CO₂ and 95% air. After 9 days, colonies comprising of more than 50 cells were counted. The colonies represented precursor cells of the granulocyte/macrophage lineage (huGM-CFC). Survival was expressed as a % of the number of colonies at zero dose temozolomide.

### Xenograft studies

### Animals

BALB-C derived athymic male mice (nu/nu athymic) weighing between 25-35g were obtained from the in-house breeding colony of the Paterson Institute for Cancer Research, Christie Hospital NHS Trust, Wilmslow Road, Manchester M20 9BX, England (ASU mice). The animals were housed in a sterile environment until required for experiments. In one experiment mice were obtained from Harlan-Olac, Harlan UK Limited, Shaw's Farm, Blackthorn, Bicester, Oxon. 0X6 OPT, England (OLAC mice). These mice weighed between 17 - 23g and were subsequently found to be more sensitive to the toxic effects of the combination (inactivator and BCNU) treatment. For this reason lower doses were administered.

### Cells

A375M (human melanoma) cells were grown in DMEM containing 10% fetal bovine serum. The cells were prepared for in vivo inoculation by incubation with 0.01% trypsin.

### Tumours

Cells (10⁶) in 100µl of PBS were injected subcutaneously into the right-hand flank of 8-10 week old nu/nu athymic mice. These cells were allowed to develop into a tumour for 3-4 weeks for passaging into experimental animals. Tumour blocks measuring 2mm x 2mm x 2mm were implanted subcutaneously on the right hand flank. These animals were ready for use in inactivator experiments in 7-10 days.

### Drug Treatment

Nu/Nu mice were treated with either 30 or 60 mg/kg 0⁶-benzylguanine, or B.4205 and the appropriate vehicle control (i.p), 60 - 90 minutes prior to 10 - 25 mg/kg BCNU (i.p.). 0⁶-benzylguanine and B.4205 were prepared as a 5 mg/kg solution in corn oil and BCNU (2mg/ml) in PBS + 3% ethanol.

### Tumour Measurements

Xenograft tumour measurements were taken every 1-2 days by workers using digital calipers. Tumour volume was calculated using the formula (h x w x 1) π/6. The experimental animals were also weighed every 1-2 days. Measurements continued until tumours in the control animals reached the maximum allowable volume (i.e. 1cm x 1cm x 1cm).

### RESULTS

Tables 4, 5 and 6 shows the physical, biochemical and in vivo data for each of the inactivators. The tests listed are explained in the Methods section.

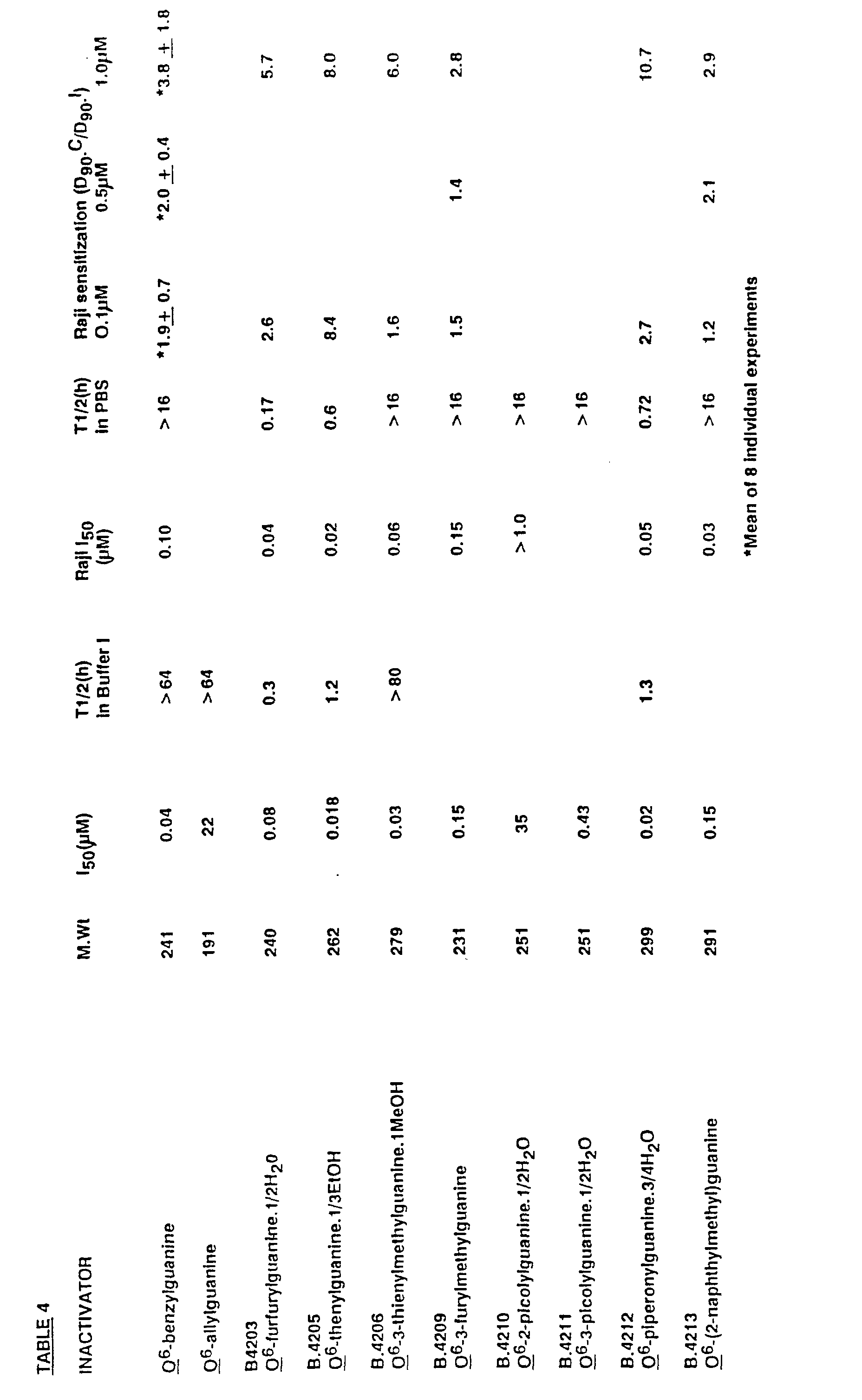

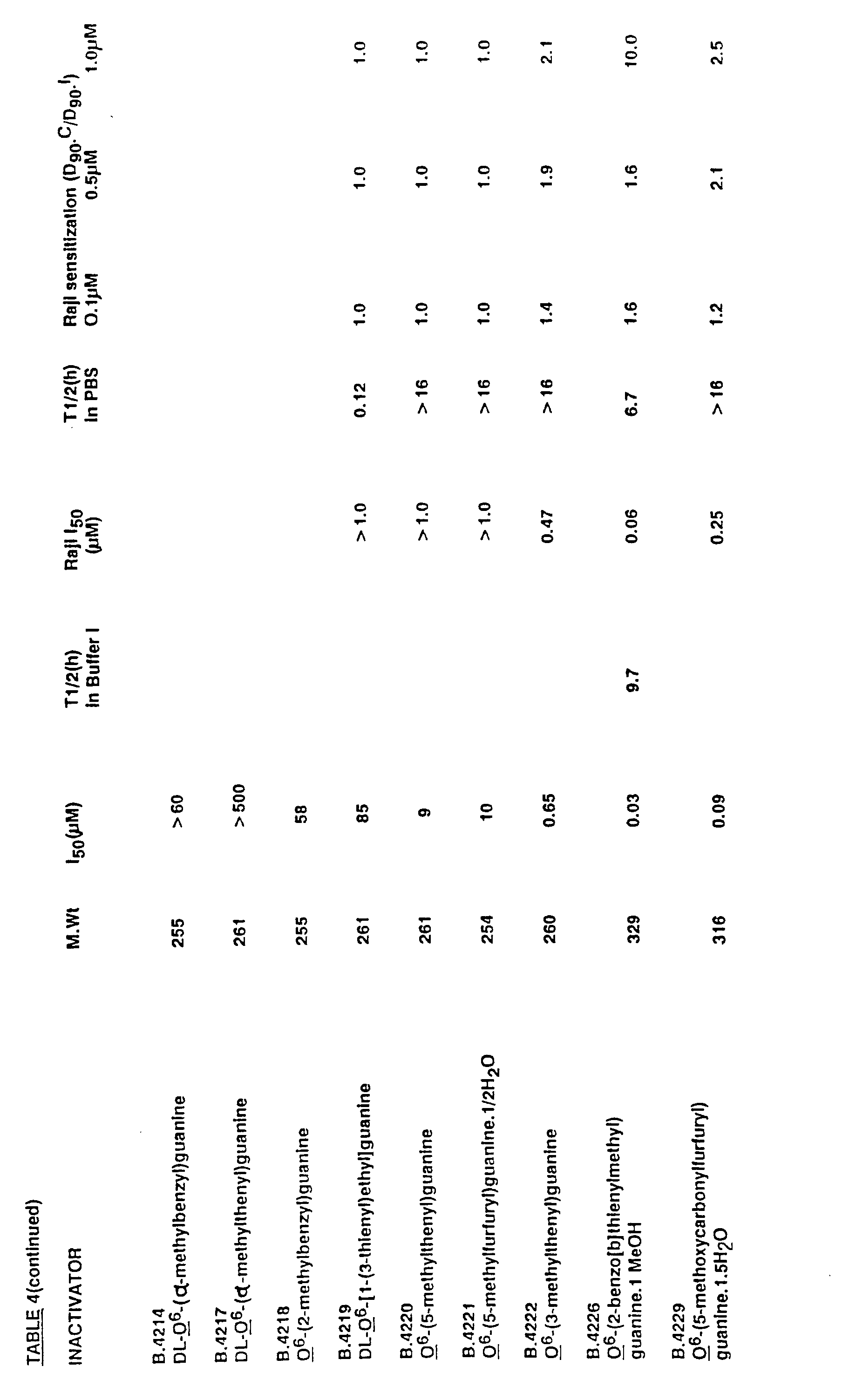

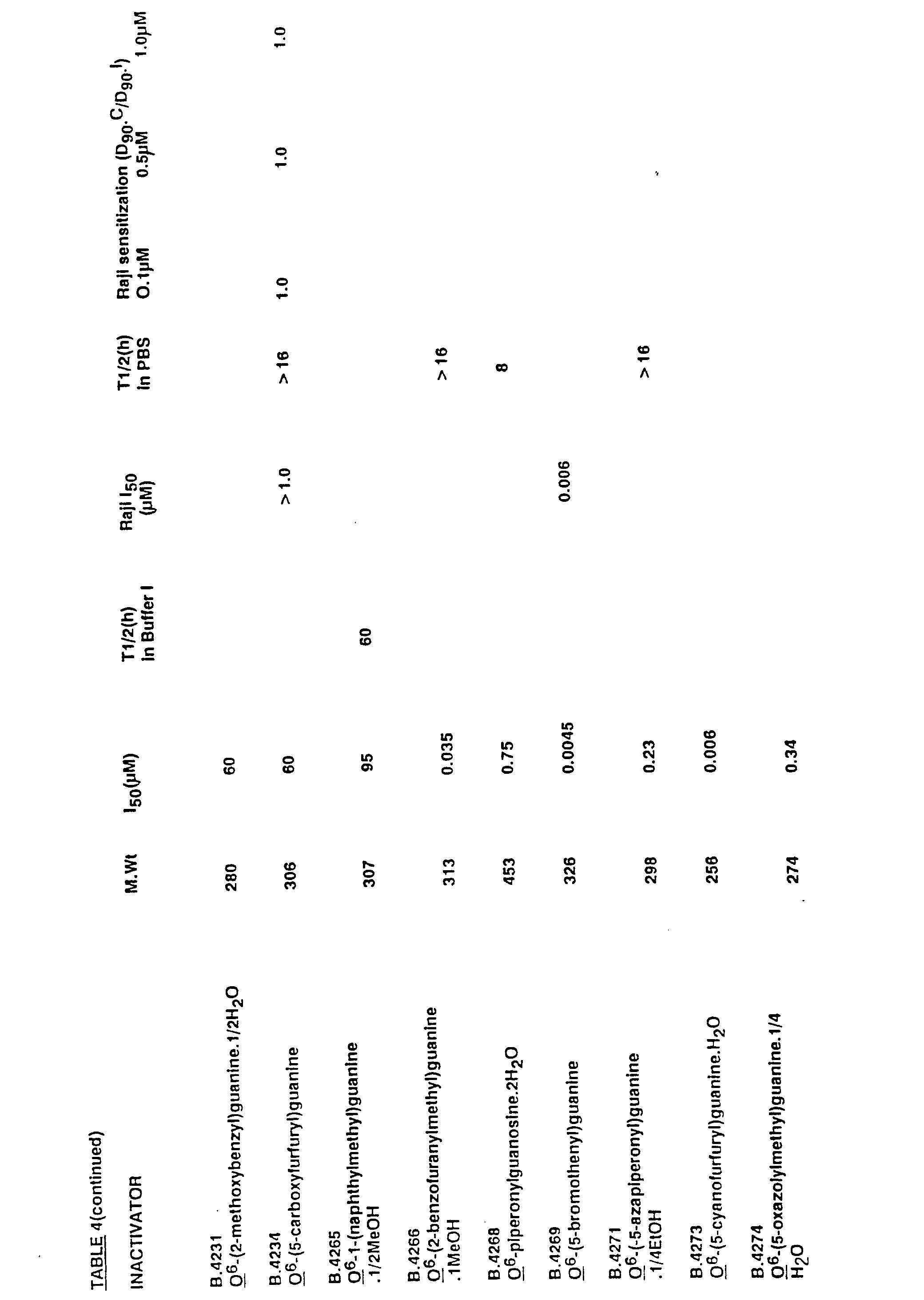

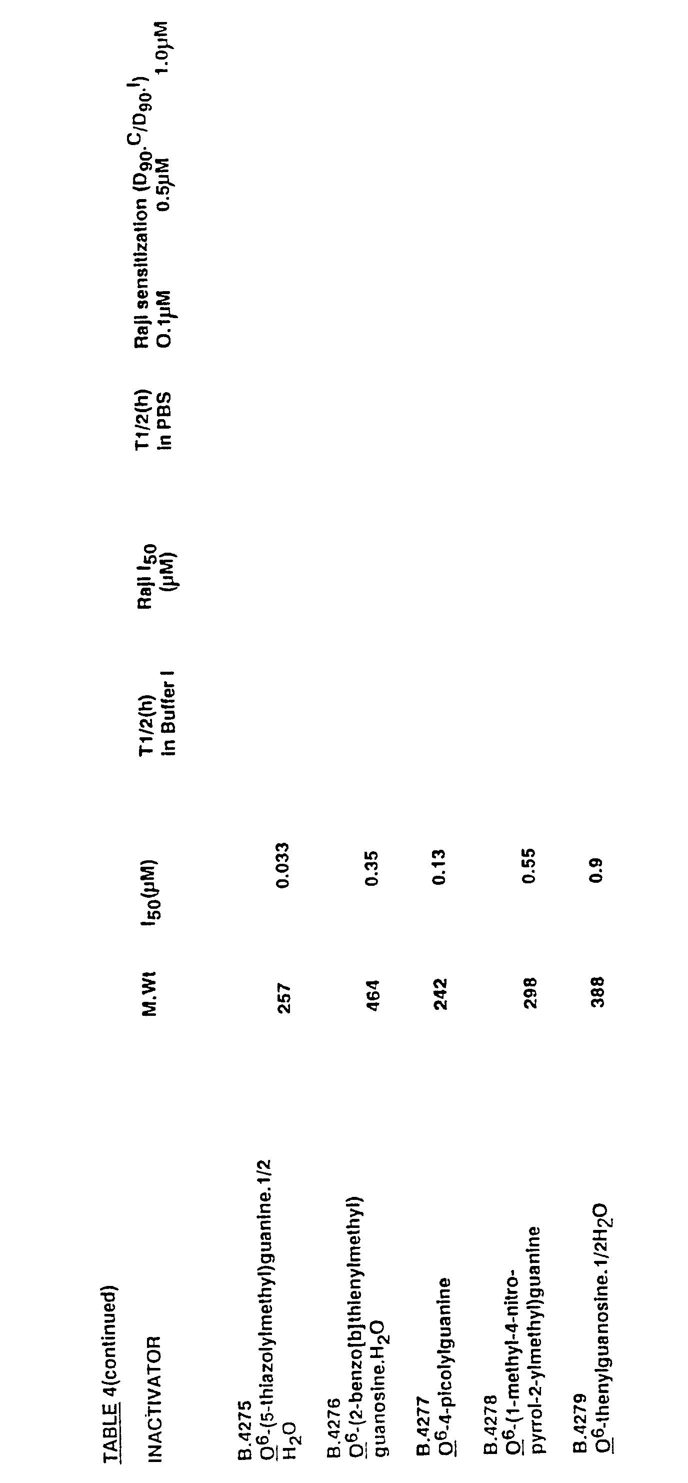

**Table 5**

| Inactivation of ATase in Mammalian Cells I₅₀(µM) | | | | | |
|---|---|---|---|---|---|
| **INACTIVATOR** | **Hamster + 120** | **CELL LINE Rat Yoshida** | **Human XP** | **Human Raji** | **Human WiDr** |
| 0⁶-benzylguanine | 0.20 | 0.14 | 0.07 | 0.10 | >0.09 |
| B.4203 | 0.12 | 0.06 | 0.06 | 0.04 | 0.08 |
| B.4205 | 0.03 | 0.02 | 0.03 | 0.02 | 0.02 |

**Table 6**

| **A375M sensitization (D**_{**50**}**.**^{**C**}**/D**_{**50**}**.**^{**I**}**)** | | | |
|---|---|---|---|
| **INACTIVATOR** | **0.5uM** | **1.0uM** | **5.0uM** |
| 0⁶-benzylguanine | 3.1 | 2.3 | 4.2 |
| 0⁶-allylguanine | 1.3 | | 1.9 |
| B.4203 | 2.0 | | 2.5 |
| B.4205 | 2.8 | 2.3 | 3.5 |
| B.4206 | 3.3 | | 4.6 |
| B.4209 | | 2.5 | |
| B.4210 | | 1.5 | |
| B.4212 | 2.3 | | 2.5 |
| B.4213 | | 2.0 | |
| B.4220 | | 1.3 | |
| B.4221 | | 1.0 | |
| B.4222 | | 1.4 | |
| B.4226 | 3.8 | | 4.5 |
| B.4229 | | 1.8 | |
| B.4234 | | 0.8 | |
| B.4266 | 6.3 | | 6.3 |

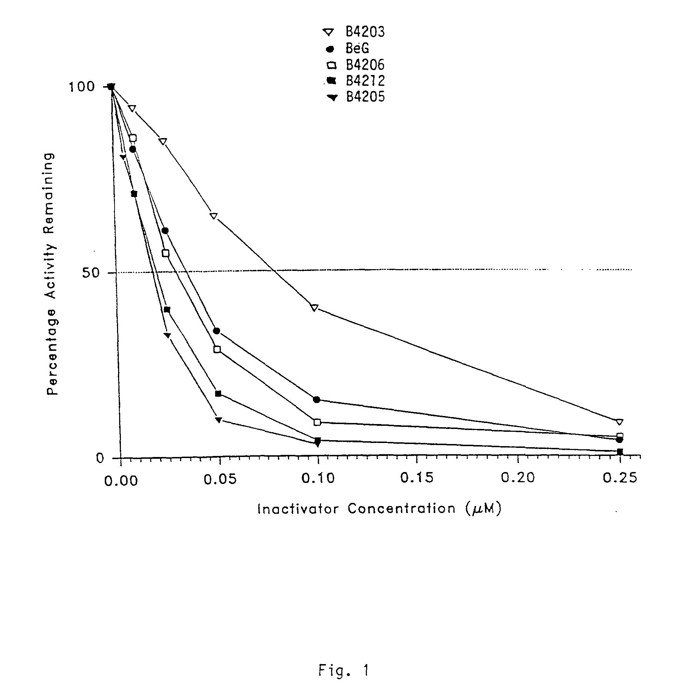

Figure 1 shows the result of the in vitro ATase inactivation assay using 4 of the compounds. B.4206 was somewhat more effective than BeG but B.4212 and B.4205 were considerably better under the assay conditions used. B.4203 was not as effective as BeG.

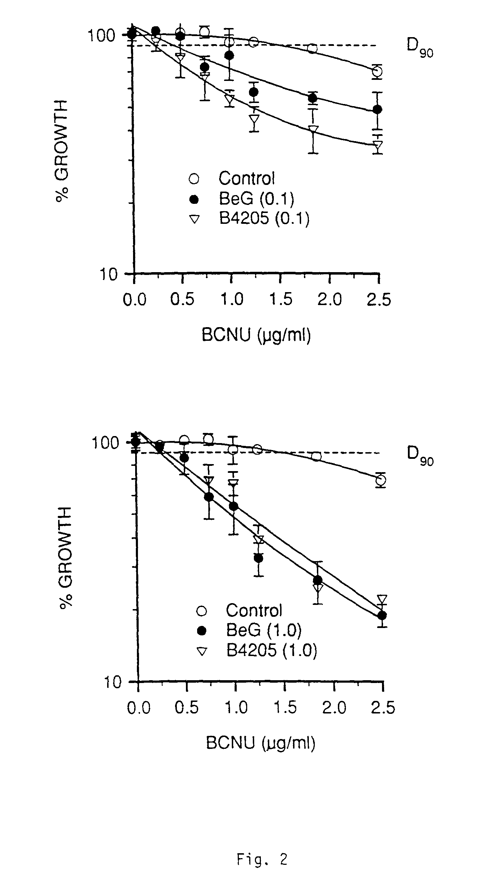

Figure 2 shows that at 0.1uM inactivator, B.4205 was more effective than BeG in sensitizing Raji cells to the growth inhibitory effects of BCNU: at 1.0uM inactivator, B.4205 and BeG were equally effective in this respect.

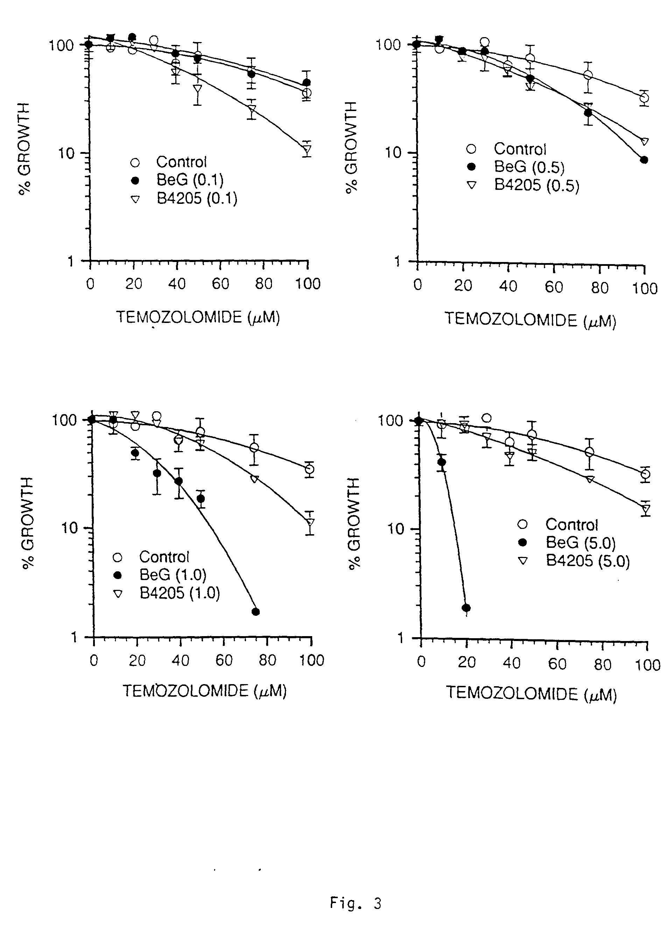

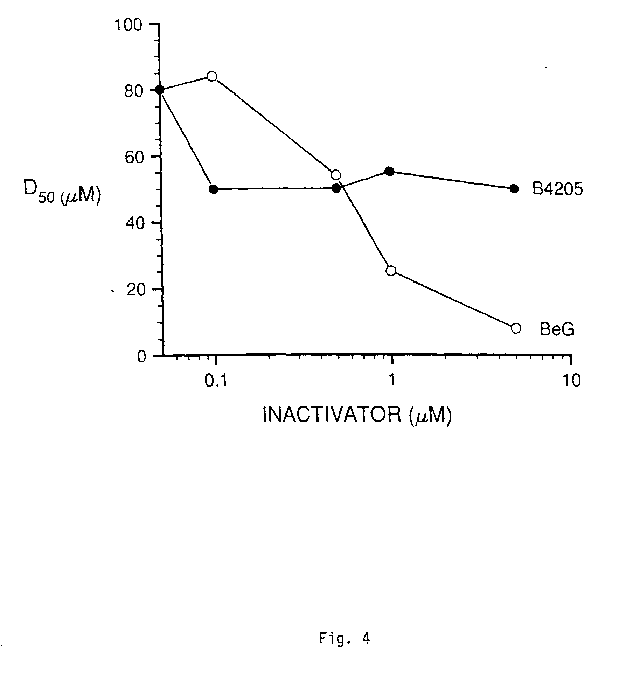

Figure 3 shows that at 0.1uM inactivator, B.4205 was more effective than BeG in sensitising Raji cells to the growth inhibitory effects of temozolomide but that as the doses of the inactivators were increased, sensitization by BeG became more effective whilst that by B.4205 remained the same. This lack of dose response with B.4205 but clear dose response with BeG is shown more clearly in Figure 4.

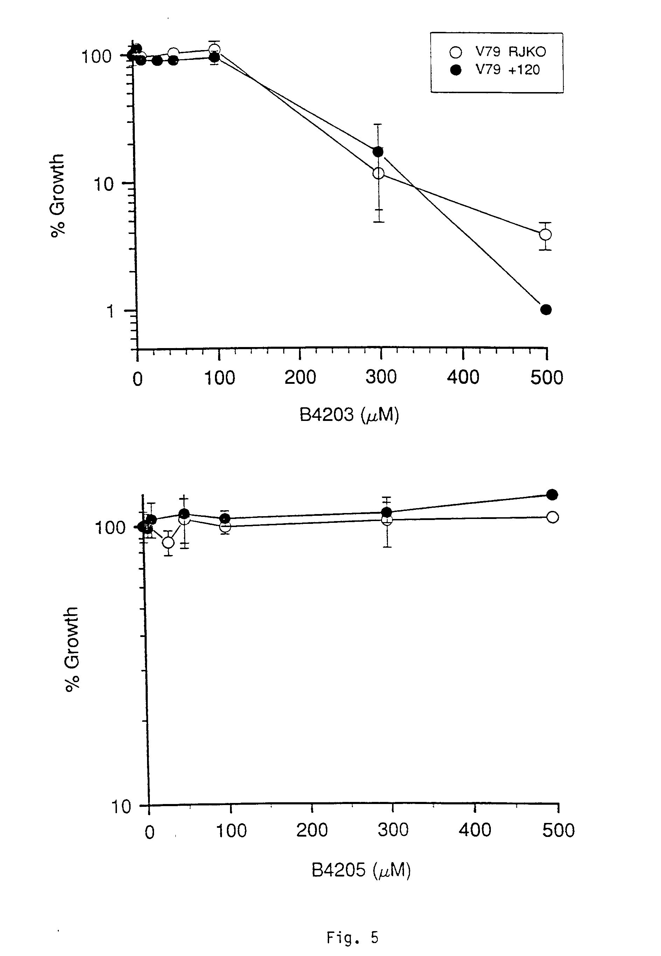

Figure 5 shows that whilst some growth inhibition of V79 cells was produced by B.4203 at doses in excess of 100uM (i.e. at least 100x higher than the I₅₀ dose for this compound ), no such effects were seen with B.4205 up to the maximum concentration used.

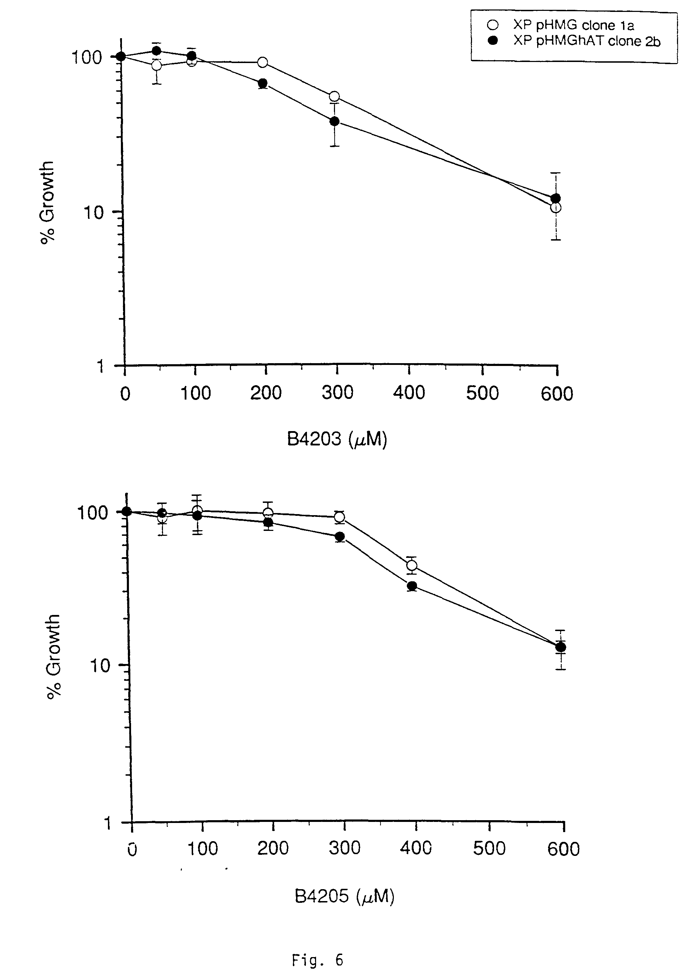

Figure 6 shows that XP cells were as susceptible to the growth inhibitory effects of B.4203 but that these cells were more sensitive than V79 cells to the effects of B.4205. However, the doses required for growth inhibition were at least 100 times that required for ATase inactivation. It can be concluded that the inherent growth inhibitory effects of these inactivators would not contribute detectably to the sensitization of cells to the growth inhibitory effects of the alkylating agents.

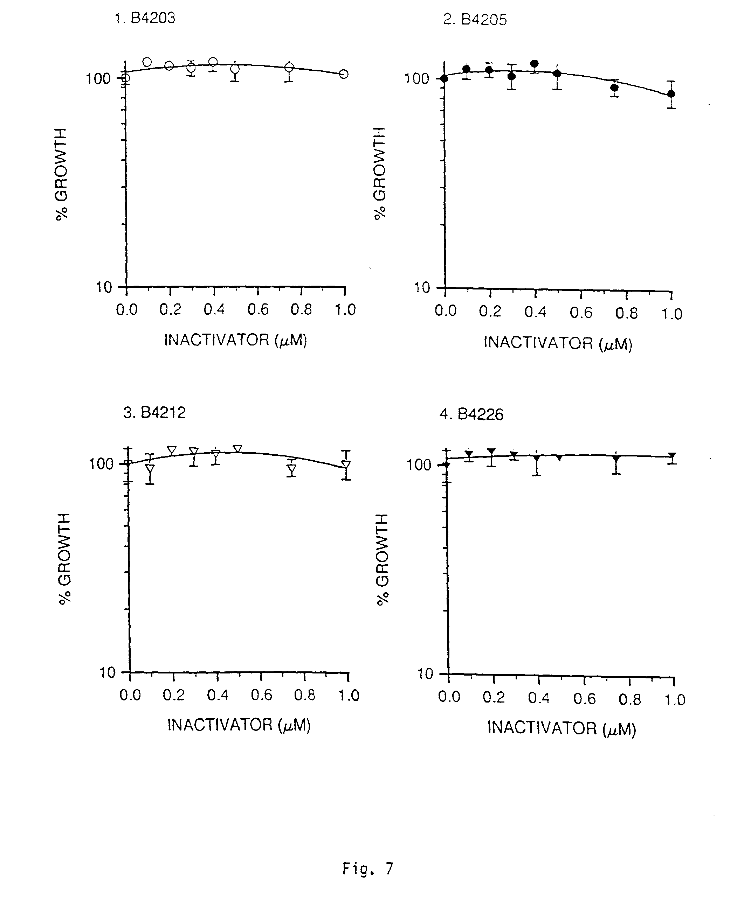

Figure 7 shows that no substantial growth inhibitory effects were produced in Raji cells when the given compounds were allowed to undergo hydrolysis (see Methods) before being added to the cells without further addition of alkylating agents. Under these experimental conditions therefore, the decomposition products of the agents would not be expected to contribute to the growth inhibitions seen using combinations of these agents and alkylating agents.

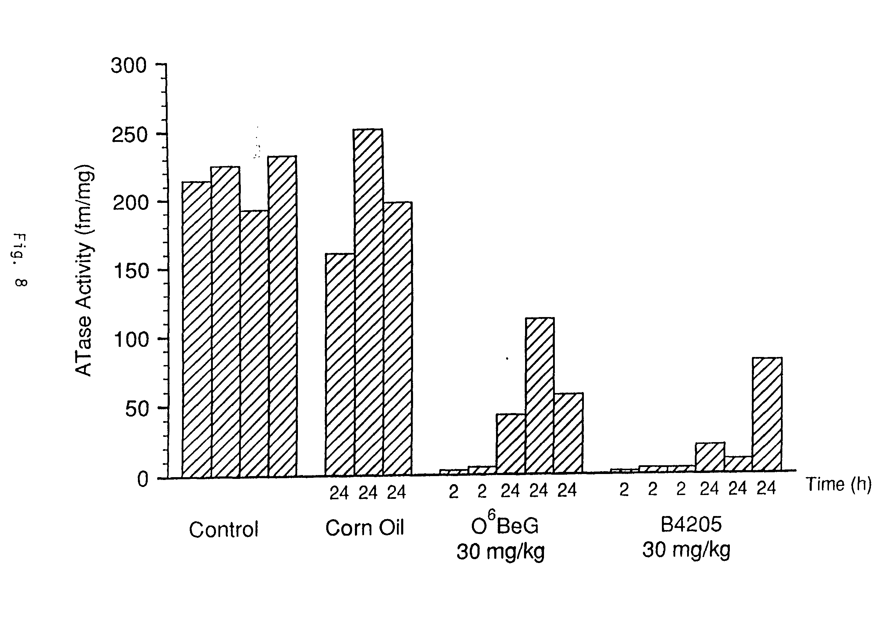

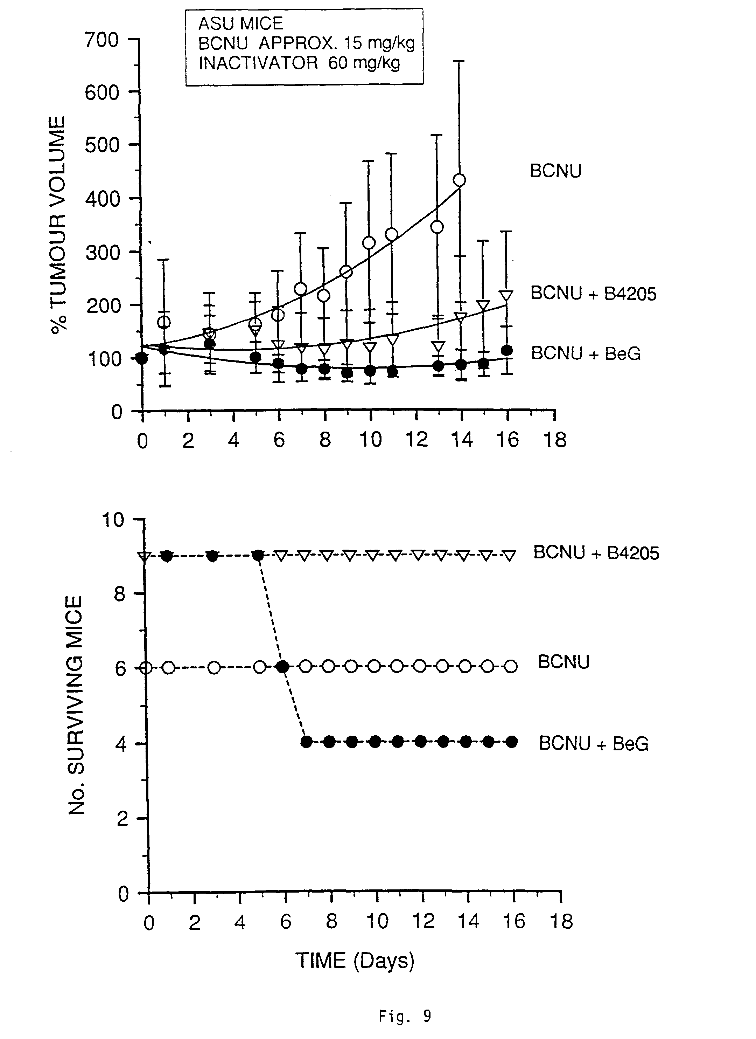

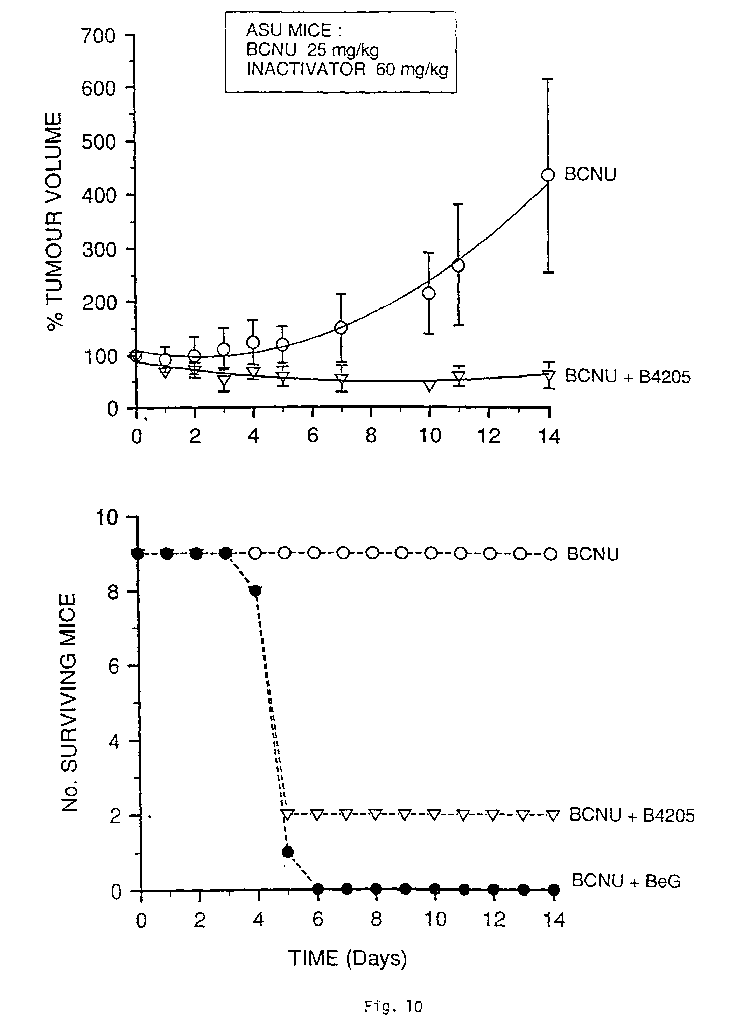

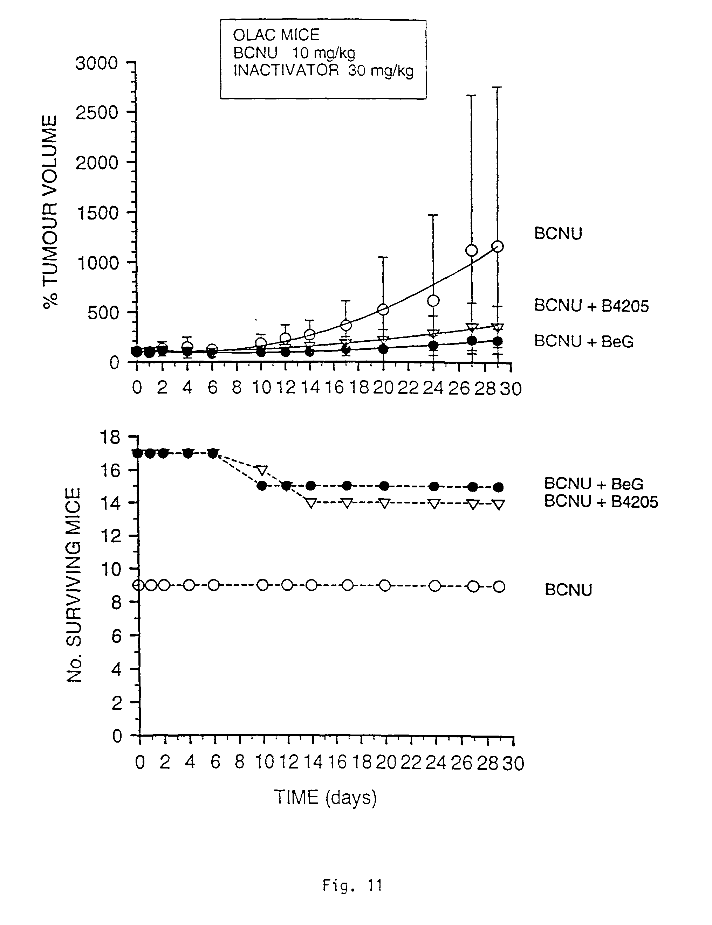

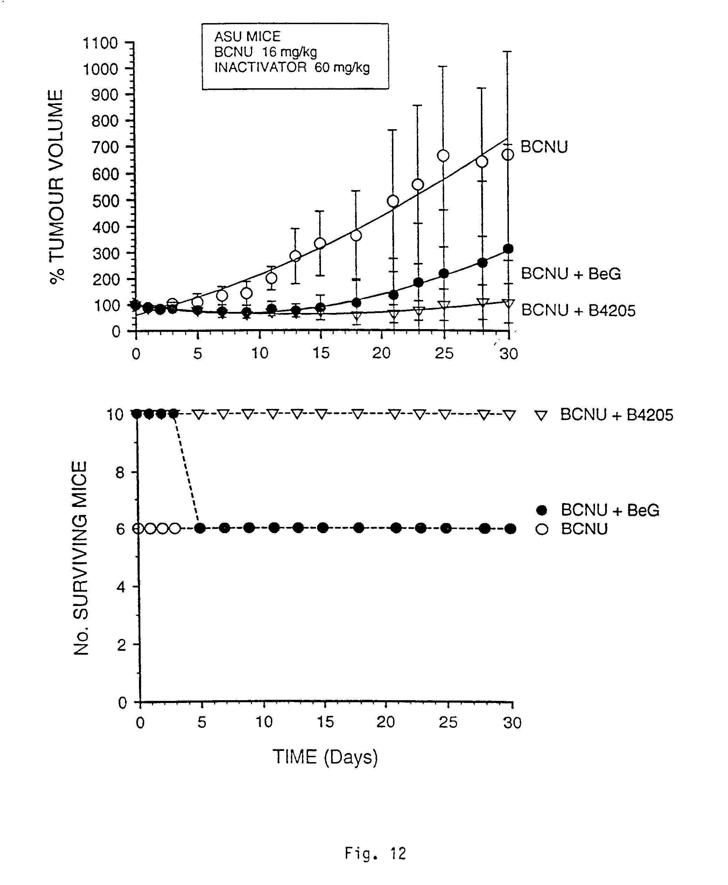

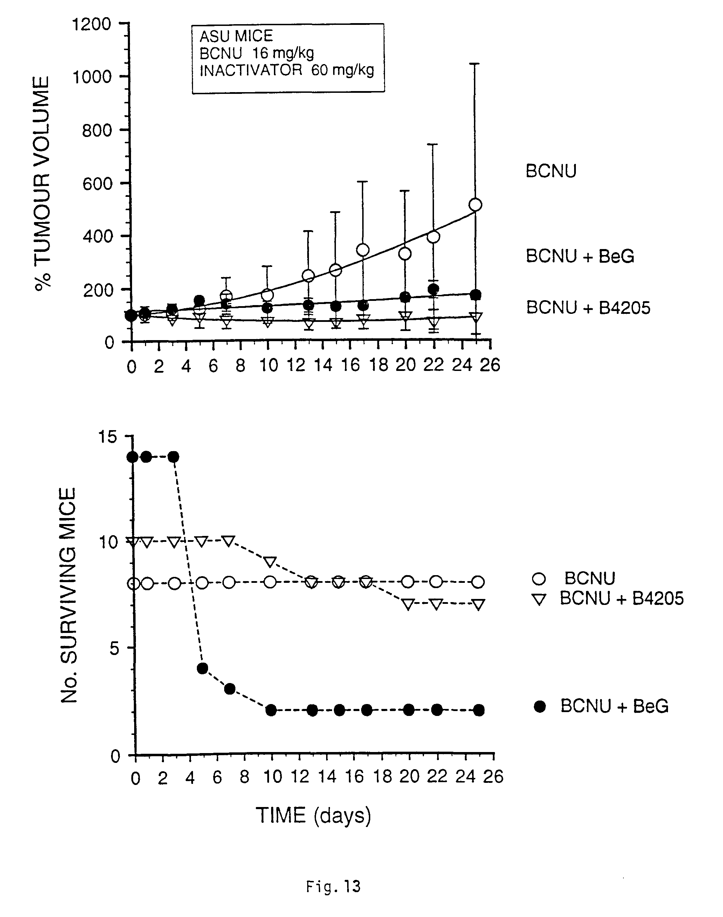

Figure 8 - 13 shows the results of the xenograft study in greater detail. The depletion and recovery of ATase activity following exposure to 0⁶-benzylguanine or B.4205 was measured in A375 tumour xenograft (Fig.8) extracts prepared from tissues of animals sacrificed 2 or 24 hours after administration of inactivator. Figures 9-13 indicate the sensitization of A375 tumour xenografts in nude mice to either 0⁶-benzylguanine or B.4205 in combination with BCNU as described in the Methods section. In each Figure the top graph shows the percentage increase in tumour volume over the time course of the experiment. The lower graph shows the number of animals in each treatment group and the number surviving following treatment. The graphs show that B.4205 is comparable to 0⁶-benzylguanine (BeG) in reducing tumour volume and is substantially less toxic in combination with BCNU than BeG in combination with BCNU. In human patients, cutaneous malignant melanoma is treated with BCNU, particularly in the USA (Balch, C.M., Houghton, A. & Peters, L. (1989) "Cutaneous Melanoma" in Cancer: Principles and Practise of Oncology, De Vita, V.T., Helman, S. & Rosenberg, S.A. (eds), pp1499-1542, Lipincott: Philadelphia) so that the human melanoma xenograft grown in nude mice is an animal model system that is clinically highly relevant.

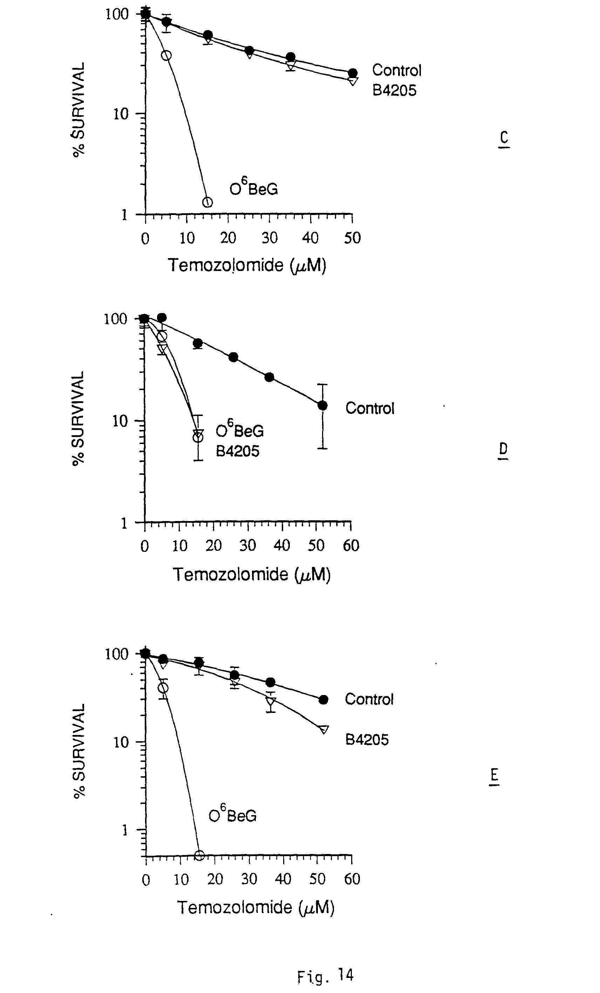

Figure 14 shows the results of the tests on sensitization of human bone marrow cells to temozolomide as described in the Methods section above. The bone marrow cells were obtained from three patients identified as C, D and E respectively. The survival curves shown relate to the cytotoxic effect of combined inactivator/temozolomide treatment. It is desirable that this effect should be reduced. The results for patients C & E show that B.4205 had a smaller sensitization effect than 0⁶-benzylguanine (0⁶BeG); for patient D there was no differential but this result is regarded as a reasonable variation in scientific testing with human material.
Table 7 shows the toxic effect of inactivator alone on bone marrow cells obtained from 5 patients A - E, including the same patients C - E as in Figure 17. The results for B.4205 are comparable to those for 0⁶-benzylguanine. Note that the cells of patient B were almost twice as sensitive to both BeG and B.4205 in comparison to the other samples.

**Table 7**

| INACTIVATOR (10uM) TOXICITY IN BONE MARROW CELLS | | | |
|---|---|---|---|
| Patient | Date of Experiment | No. of colonies as % of control (ie no inactivator) | |
| | | O⁶-benzylguanine | B.4205 |
| A | 05/02/93 | 95 | 91 |
| B | 25/03/93 | 46 | 46 |
| C | 17/05/93 | 88 | 97 |
| D | 05/05/93 | 88 | 118 |
| E | 05/05/93 | 89 | 89 |

### Summary of Findings

1) All of the compounds have been tested for their ability to inactivate recombinant human ATase under standard conditions in an in vitro assay. Under these conditions two of the compounds (B.4214 and B.4217) did not inactivate ATase up to the highest concentration used but these were compounds in which R' is methyl. The remainder of the compounds inactivated ATase with I₅₀ values ranging from 0.0045 to 95 µmolar. Eight compounds (B.4205, B.4206, B.4212, B.4226, B.4266, B.4269, B.4273, B.4275) had I₅₀ values lower than that of BeG (Table 4).
2) The inactivators underwent hydrolysis in aqueous solution at different rates (half lives 0.17 to>80 hours) but this was not related to their efficiency as ATase inactivators. (Table 4)
3) Compounds that were efficient in the inactivation of ATase in vitro (I₅₀ <1.0µM) also inactivated ATase in human cells with I₅₀ values that were generally only slightly (mean approx. 1.5 times) higher than those found using recombinant protein in the in vitro assay. (Table 4)
4) B.4205 inactivated ATase in human, rat and Chinese hamster cells with similar effectiveness (I₅₀ values 0.02-0.03). For B.4203 the range was 0.04-0.12. In the cell lines used B.4205 and B.4203 were up to 7 times more effective than BeG. (Table 5)
5) B.4203 and B.4205 were toxic to the XP cells studied and B.4203 was toxic to the V79 cells studied but only at concentrations that were at least 100 times higher than those at which sensitization to BCNU was observed. (Figs 5 & 6)
6) Compounds that were efficient in the inactivation of ATase in vitro (I₅₀ <1.0µM) and in Raji cells (I₅₀ <1.0µM) also sensitized Raji and A375M cells to the growth inhibitory effects of BCNU where this was tested. (Table 4)
7) At inactivator concentrations of 0.1µM, B.4205 was more effective than BeG in sensitising Raji cells to the growth inhibitory effects of temozolomide. (Fig 3)
8) The in vitro assay can be used to predict which compounds are most likely to be effective sensitisers of mammalian cells to the growth inhibitory effects of BCNU and related cytotoxic agents.
9) B.4205 was similar or slightly more effective than BeG in sensitising human melanoma xenografts grown in nude mice to the growth inhibitory effects of BCNU.(Figs. 9-13)
10) B.4205 was as effective as BeG in inactivating ATase in human melanoma xenografts grown in nude mice.(Fig. 8)
11) The in vitro assay and/or the xenograft ATase depletion assay may be used to predict which compounds are most likely to be effective sensitisers of melanoma xenografts to the growth inhibitory effects of BCNU and related cytotoxic agents.
12) In contrast to BeG, which caused death in up to 70% of the treated animals, B.4205 had very little effect on the sensitivity of nude mice bearing human melanoma xenografts to the acute toxic effects of BCNU under the conditions used. (Figs. 9-13)
13) BeG sensitised the GM-CFCs in the three human bone marrow samples tested to the toxic effects of temozolomide but in two of these samples, little or no sensitization was produced by B.4205. This assay may therefore be used to predict the possible myelosuppressive effects of ATase inactivators when used in the clinic in combination with BCNU and related agents. (Fig. 14)

### References

- 1.: W.N. Haworth and W.G.M. Jones, J.Chem.Soc., 1944, 667
- 2.: T. Reichstein, Helv.Chim.Acta, 9, 1926, 1066.
- 3.: V. Bocchi, G. Casnati, A. Dossena and R. Marchelli, Synthesis, 1979, 961.
- 4.: P.A. Finan, J.Chem.Soc., 1963, 3917; J.A. Moore and J.E. Kelly, J. Polym.Sci., Polym.Chem.Ed., 22, 1984, 863.
- 5.: J. Kiermayer, Chemiker-Zeitung, 19, 1895, 1004.
- 6.: A. Carotti, F. Campagna and R. Ballini, Synthesis, 1979, 56.
- 7.: A.J. Floyd, R.G. Kinsman, Y. Roshan-Ali and D.W. Brown, Tetrahedron, 39, 1983, 3881
- 8.: V.T. Suu, N.P. Buu-Hoi and N.D. Xuong, Bull.Soc.Chim.France, 1962, 1875.
- 9.: T. Reichstein and L. Reichstein Helv.Chim.Acta, 13, 1930, 1275.
- 10.: H. Takeshita, H. Mametsuka and H. Motomura. J. Heterocycl.Chim., 23, 1986, 1211.
- 11.: F.J. Wolf and J. Weijiard, Org.Synth.,Coll.Vol.4, 1963, 124.
- 12.: A.M. van Leusen, B.E. Hoogenboom and H. Siderius, Tetrahedron Lett., 1972, 2369.
- 13.: A. Maquestiau, R. Flammang and F.B. Abdelouahab, Heterocycles, 29, 1989, 103.
- 14.: S. Fallab, Helv.Chim.Acta, 35, 1952, 215
- 15.: A.S. Kende, K. Kawamura and R.J. DeVita, J.Amer.Chem.Soc., 112, 1990, 4070.
- 16.: S. Trofimenko, J.Org.Chem., 28, 1963, 3243.
- 17.: I. Sawhney and J.R.H. Wilson (Shell Internationale), EP. 395,174, 1989 (Chem.Abs., 114, 143410s).
- 18.: F. Dallacker, P. Fechter and V. Mues, Z. Naturforsch., 34b, 1979, 1729.
- 19.: P. Fournari, Bull.Soc.Chim.France, 1963, 488.
- 20.: L. Grehn, Chem.Scr., 16, 1980, 72.
- 21.: Morten, J.E.N. & Margison, G.P. (1988) Carcinogenesis 9, 45-49
- 22.: Scudiero, D.A., Shoemaker R.J., Paull K.D., Monks A., Tierney S., Nofziger T.H., Currens M.J., Seniff D, & Boyd M.R. (1988), Cancer Research 48, 4827-4833
- 23.: Fan, C.-Y., Potter, P.M., Rafferty, J.A., Watson, A.J., Cawkwell, L., Searle, P.F., O'Connor, P.J. and Margison, G.P. (1991) Nucleic Acids Res. 18, 5723-5727
- 24.: Carmichael J., DeGraff W.G., Gazdar A.F., Minna J.D., Mitchell J.B. (1987). Evaluation of a tetrazolium based semiautomated colorimetric assay : assessment of chemosensitivity testing. Cancer Res. 47 : 936-942.
- 25.: Wilkinson, M.C., Potter, P.M., Cawkwell, L., Georgiadis, P., Patel, D., Swann, P.F. and Margison, G.P. (1989) Nucleic Acids Res. **17**, 8475-8484.
- 26: Wilkinson, M.C., Cooper, D.P., Southan, C., Potter, P.M. & Margison, G.P. (1990) Nucleic Acids Res., **18**, 13-16.

In the specification the abbreviations "1h" or "2h" etc. mean "1 hour", "2 hours", etc.

## Claims

1. O⁶-alkylguanine derivatives of the formula I: 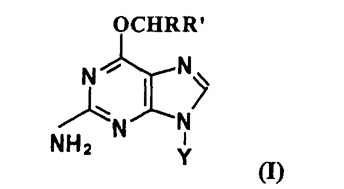 wherein
Y is H, ribosyl, deoxyribosyl, or R"XCHR''', wherein X is O or S, R" and R"' are C₁-C₂₀alkyl, or substituted derivatives thereof having substitution by one or more of hydroxy, C₁-C₂₀alkoxy, amino, C₁-C₂₀alkylamino, amido or ureido;
R' is H or C₁-C₂₀alkyl or hydroxyC₁-C₂₀alkyl;
R is
(i) a cyclic group having at least one 5- or 6-membered heterocyclic ring, optionally with a carbocyclic or heterocyclic ring fused thereto, the or each heterocyclic ring having at least one hetero atom chosen from O, N, or S, or a substituted derivative thereof having substitution in the heterocyclic ring(s) and/or carbocyclic ring(s) by one or more of C₁-C₂₀alkyl, C₁-C₂₀ alkenyl, C₁-C₂₀alkynyl, halo, haloC₁-C₂₀alkyl, nitro, cyano, hydroxyC₁-C₂₀alkyl, SOₙR""where R"" is C₁-C₂₀alkyl and n = 0, 1 or 2, or a carboxyl or ester group of the formula - COOR⁵ wherein R⁵ is H or C₁-C₂₀alkyl; or
(ii) naphthyl or a substituted derivative thereof having substitution by one or more of C₁-C₂₀alkyl, C₁-C₂₀alkenyl, C₁-C₂₀alkynyl, halo, haloC₁-C₂₀alkyl, nitro, cyano, hydroxyC₁-C₂₀alkyl, SOₙR"" where R"" is C₁-C₂₀ alkyl and n = 0, 1 or 2, or a carboxyl or ester group of the formula - COOR⁵ wherein R⁵ is H or C₁-C₂₀alkyl;
and pharmaceutically acceptable salts thereof.

2. A compound according to claim 1
wherein
Y is H, ribosyl, deoxyribosyl, or R"XCHR''', wherein X is O or S, R" and R''' are C₁-C₂₀alkyl, or substituted derivatives thereof having substitution by one or more of hydroxy, amino, C₁-C₂₀alkylamino, amido or ureido;
R' is as defined in claim 1;
R is
(i) a cyclic group having at least one 5- or 6-membered heterocyclic ring, optionally with a carbocyclic or heterocyclic ring fused thereto, the or each heterocyclic ring having at last one hetero atom chosen from O, N, or S, or a substituted derivative thereof having substitution in the heterocyclic ring(s) and/or carbocyclic ring(s) by one or more of C₁-C₂₀alkyl, C₁-C₂₀alkenyl, C₁-C₂₀alkynyl, halo, haloC₁-C₂₀alkyl, nitro, cyano, hydroxyC₁-C₂₀alkyl, SOₙR"" where R"" is C₁-C₂₀alkyl and n = 0, 1 or 2; or
(ii) naphthyl or a substituted derivative thereof having substitution by one or more of C₁-C₂₀alkyl, C₁-C₂₀alkenyl, C₁-C₂₀alkynyl, halo, haloC₁-C₂₀alkyl, nitro, cyano, hydroxyC₁-C₂₀alkyl, SOₙR"" where R"" is C₁-C₂₀ alkyl and n = 0, 1 or 2.
and pharmaceutically acceptable salts thereof.

3. A compound according or claim 1 or 2 wherein R is:
(a) a 5- or 6-membered heterocyclic ring, or
(b) a benzo derivative thereof, the O⁶-alkylguanine moiety being attached to R at either the heterocyclic or the benzene ring.

4. A compound according to claim 1 or 2 wherein R is a 5-membered heterocyclic ring, having at least one S atom therein, or a substituted derivative thereof as defined in claim 1 or 2 respectively.

5. A compound according to claim 1 or 2 wherein R is a thiophene ring, or a substituted derivative thereof as defined in claim 1 or 2 respectively.

6. A compound according to claim 5 wherein R is a thiophene ring substituted in the ring by halo.

7. A compound according to claim 6 wherein halo is iodo, bromo, chloro or fluro.

8. A compound according to claim 1 or 2 wherein R is a furan ring, or a substituted derivative thereof as defined in claim 1 or 2 respectively.

9. A compound according to claim 1 or 2 wherein R includes a heterocyclic and/or carbocyclic ring substituted by halo, haloC₁-C₂₀alkyl, cyano, SOₙR"" where R"" is C₁-C₂₀alkyl and n = 0, 1 or 2, or -COOR⁵ wherein R⁵ is C₁-C₂₀alkyl.

10. A compound according to claim 1 or 2 wherein R includes a heterocyclic ring substituted by halo, cyano or an ester group of the formula -COOR⁵ wherein R⁵ is C₁-C₂₀alkyl.

11. A compound according to any of the preceding claims wherein R' is H.

12. A compound according to claim 1 or 2 which is O⁶-thenylguanine or a substituted derivative thereof having substitution in the thiophene ring by a halo, cyano or ester group.

13. A compound according to claim 12 which is O⁶-thenylguanine.

14. A compound according to claim 12 which is O⁶-thenylguanine having substitution in the thiophene ring by halo.

15. A compound according to claim 14 wherein halo is iodo, bromo, chloro or fluoro.

16. A compound according to claim 1 which is O⁶-(3-thienylmethyl)guanine.

17. A compound according to claim 1 which is O⁶-piperonylguanine.

18. A compound according to claim 1 which is O⁶-furfurylguanine.

19. A compound according to claim 1 which is O⁶-(3-furylmethyl)guanine.

20. A compound according to claim 1 which is O⁶-(2-benzo[b]thienylmethyl)guanine.

21. A compound according to claim 1 which is O⁶-(2-benzofuranylmethyl)guanine.

22. A compound according to claim 1 which is O⁶-(5-thiazolylmethyl)guanine.

23. A compound according to claim 1 which is O⁶-(5-methoxycarbonylfurfuryl)guanine.

24. A compound according to claim 1 which is O⁶-(5-bromothenyl)guanine.

25. A compound according to claim 1 which is O⁶-(5-cyanofurfuryl)guanine.

26. A compound according to claim 1 which is O⁶-(2-benzo[b]thienylmethyl)guanosine.

27. A compound according to claim 1 which is O⁶-(4-picolyl)guanine.

28. A compound according to claim 1 which is O⁶-(2-naphthylmethyl)guanine.

29. A pharmaceutical composition comprising a compound according to any of the preceding claims and a pharmaceutically acceptable excipient.

30. A pharmaceutical composition according to claim 29 further comprising an alkylating agent.

31. A compound according to any of claims 1 to 28 for use in a method for depleting O⁶-alkylguanine-DNA alkyltransferase activity in a host.

32. Use of a compound according to any of claims 1 to 28, in the manufacture of a medicament for depleting O⁶-alkylguanine-DNA alkyltransferase activity in tumour cells.

33. Use of a compound according to any of claims 1 to 28 and an alkylating agent in the manufacture of a one- or two-part pharmaceutical composition for treating tumour cells.

34. Use according to claim 32 or 33 wherein the compound according to any of claims 1 to 28 is O⁶-thenylguanine or a substituted derivative thereof having substitution in the thiophene ring by a halo, cyano or ester group of the formula - COOR⁵ wherein R⁵ is C₁-C₂₀alkyl.

35. A composition according to claim 30 wherein the alkylating agent is selected from 1, 3 bis (2-chloroethyl)-1-nitrosourea (BCNU) and temozolomide.

36. Use according to any of claims 32 to 34 wherein the alkylating agent is selected from 1, 3 bis (2-chloroethyl)-1-nitrosourea (BCNU) and temozolomide.

37. A process for preparing a compound of the formula I as claimed in claim 1 which comprises reacting sodium hydride with a solution of RR'CHOH (wherein R and R' are as defined in claim 1) in an organic solvent, adding 2-amino-N,N,N-trimethyl-1H-purin-6-aminium chloride or 2-amino-6-chloropurine riboside, treatment with weak acid and ether, and extracting the desired product.

## Patentansprüche

1. O⁶-Alkylguaninderivate der Formel I: 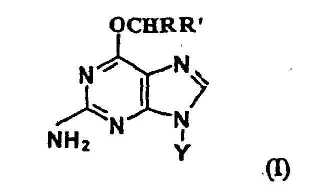 wobei
Y = H, Ribosyl, Desoxyribosyl oder R"XCHR''' ist, wobei X = O oder S ist, R" und R"' C₁-C₂₀-Alkyl oder substituierte Derivate davon mit Substitution durch eine oder mehrere Hydroxy-, C₁-C₂₀-Alkoxy-, Amino-, C₁-C₂₀-Alkylamino-, Amido- oder Ureidogruppen sind;
R' = H oder C₁-C₂₀-Alkyl oder Hydroxy-C₁-C₂₀-alkyl ist;
R folgendes ist:
(i) eine cyclische Gruppe mit wenigstens einem fünf- oder sechsgliedrigen heterocyclischen Ring, wobei gegebenenfalls ein carbocyclischer oder heterocyclischer Ring daran kondensiert ist, wobei der bzw. jeder heterocyclische Ring wenigstens ein Heteroatom aufweist, das aus O, N oder S ausgewählt ist, oder ein substituiertes Derivat davon mit Substitution in dem bzw. den heterocyclischen Ringen und/oder in dem bzw. den carbocyclischen Ringen durch eine oder mehrere der Gruppen C₁-C₂₀-Alkyl, C₁-C₂₀-Alkenyl, C₁-C₂₀-Alkinyl, Halogen, Halogen-C₁-C₂₀-alkyl, Nitro, Cyan, Hydroxy-C₁-C₂₀-alkyl, SOₙR"", wobei R"" = C₁-C₂₀-Alkyl ist und n = 0, 1 oder 2 ist, oder durch eine Carboxy- oder Estergruppe der Formel -COOR⁵, wobei R⁵ = H oder C₁-C₂₀-Alkyl ist; oder
(ii) Naphthyl oder ein substituiertes Derivat davon mit Substitution durch eine oder mehrere der Gruppen C₁-C₂₀-Alkyl, C₁-C₂₀-Alkenyl, C₁-C₂₀-Alkinyl, Halogen, Halogen-C₁-C₂₀-alkyl, Nitro, Cyan, Hydroxy-C₁-C₂₀-alkyl, SOₙR"", wobei R"" = C₁-C₂₀-Alkyl ist und n = 0, 1 oder 2 ist, oder durch eine Carboxy- oder Estergruppe der Formel -COOR⁵, wobei R⁵ = H oder C₁-C₂₀-Alkyl ist;
sowie pharmazeutisch annehmbare Salze davon.

2. Verbindung gemäß Anspruch 1, wobei
Y = H, Ribosyl, Desoxyribosyl oder R"XCHR''' ist, wobei X = O oder S ist, R" und R"' C₁-C₂₀-Alkyl oder substituierte Derivate davon mit Substitution durch eine oder mehrere Hydroxy-, Amino-, C₁-C₂₀-Alkylamino-, Amidooder Ureidogruppen sind;
R' wie in Anspruch 1 definiert ist;
R folgendes ist:
(i) eine cyclische Gruppe mit wenigstens einem fünf- oder sechsgliedrigen heterocyclischen Ring, wobei gegebenenfalls ein carbocyclischer oder heterocyclischer Ring daran kondensiert ist, wobei der bzw. jeder heterocyclische Ring wenigstens ein Heteroatom aufweist, das aus O, N oder S ausgewählt ist, oder ein substituiertes Derivat davon mit Substitution in dem bzw. den heterocyclischen Ringen und/oder in dem bzw. den carbocyclischen Ringen durch eine oder mehrere der Gruppen C₁-C₂₀-Alkyl, C₁-C₂₀-Alkenyl, C₁-C₂₀-Alkinyl, Halogen, Halogen-C₁-C₂₀-alkyl, Nitro, Cyan, Hydroxy-C₁-C₂₀-alkyl, SOₙR"", wobei R"" = C₁-C₂₀-Alkyl ist und n = 0, 1 oder 2 ist; oder
(ii) Naphthyl oder ein substituiertes Derivat davon mit Substitution durch eine oder mehrere der Gruppen C₁-C₂₀-Alkyl, C₁-C₂₀-Alkenyl, C₁-C₂₀-Alkinyl, Halogen, Halogen-C₁-C₂₀-alkyl, Nitro, Cyan, Hydroxy-C₁-C₂₀-alkyl, SOₙR"", wobei R"" = C₁-C₂₀-Alkyl ist und n = 0, 1 oder 2 ist;
sowie pharmazeutisch annehmbare Salze davon.

3. Verbindung gemäß Anspruch 1 oder 2, wobei R folgendes ist:
(a) ein fünf- oder sechsgliedriger heterocyclischer Ring; oder
(b) ein Benzoderivat davon, wobei die O⁶-Alkylguanin-Struktureinheit entweder am heterocyclischen oder am Benzolring an R gebunden ist.

4. Verbindung gemäß Anspruch 1 oder 2, wobei R ein fünfgliedriger heterocyclischer Ring mit wenigstens einem S-Atom darin oder ein substituiertes Derivat davon, wie es in Anspruch 1 oder 2 definiert ist, ist.

5. Verbindung gemäß Anspruch 1 oder 2, wobei R ein Thiophenring oder ein substituiertes Derivat davon, wie es in Anspruch 1 oder 2 definiert ist, ist.

6. Verbindung gemäß Anspruch 5, wobei R ein durch Halogen ringsubstituierter Thiophenring ist.

7. Verbindung gemäß Anspruch 6, wobei es sich bei Halogen um Iod, Brom, Chlor oder Fluor handelt.

8. Verbindung gemäß Anspruch 1 oder 2, wobei R ein Furanring oder ein substituiertes Derivat davon, wie es in Anspruch 1 oder 2 definiert ist, ist.

9. Verbindung gemäß Anspruch 1 oder 2, wobei R einen heterocyclischen und/oder carbocyclischen Ring umfasst, der durch Halogen, Halogen-C₁-C₂₀-alkyl, Cyan, SOₙR"", wobei R"" = C₁-C₂₀-Alkyl ist und n = 0, 1 oder 2 ist, oder -COOR⁵, wobei R⁵ = C₁-C₂₀-Alkyl ist, substituiert ist.

10. Verbindung gemäß Anspruch 1 oder 2, wobei R einen heterocyclischen Ring umfasst, der durch Halogen, Cyan oder eine Estergruppe der Formel -COOR⁵, wobei R⁵ = C₁-C₂₀-Alkyl ist, substituiert ist.

11. Verbindung gemäß einem der vorstehenden Ansprüche, wobei R' = H ist.

12. Verbindung gemäß Anspruch 1 oder 2, bei der es sich um 06-Thenylguanin oder ein substituiertes Derivat davon mit Substitution im Thiophenring durch eine Halogen-, Cyan- oder Estergruppe handelt.

13. Verbindung gemäß Anspruch 12, bei der es sich um O⁶-Thenylguanin handelt.

14. Verbindung gemäß Anspruch 12, bei der es sich um O⁶-Thenylguanin mit Halogensubstitution im Thiophenring handelt.

15. Verbindung gemäß Anspruch 14, wobei es sich bei Halogen um Iod, Brom, Chlor oder Fluor handelt.

16. Verbindung gemäß Anspruch 1, bei der es sich um O⁶-(3-Thienylmethyl)-guanin handelt.

17. Verbindung gemäß Anspruch 1, bei der es sich um O⁶-Piperonylguanin handelt.

18. Verbindung gemäß Anspruch 1, bei der es sich um O⁶-Furfurylguanin handelt.

19. Verbindung gemäß Anspruch 1, bei der es sich um O⁶-(3-Furylmethyl)-guanin handelt.

20. Verbindung gemäß Anspruch 1, bei der es sich um O⁶-(2-Benzo[b]thienylmethyl)guanin handelt.

21. Verbindung gemäß Anspruch 1, bei der es sich um O⁶-(2-Benzofuranylmethyl)guanin handelt.

22. Verbindung gemäß Anspruch 1, bei der es sich um O⁶-(5-Thiazolylmethyl)-guanin handelt.

23. Verbindung gemäß Anspruch 1, bei der es sich um O⁶-(5-Methoxycarbonylfurfuryl)guanin handelt.

24. Verbindung gemäß Anspruch 1, bei der es sich um O⁶-(5-Bromthenyl)-guanin handelt.

25. Verbindung gemäß Anspruch 1, bei der es sich um O⁶-(5-Cyanfurfuryl)-guanin handelt.

26. Verbindung gemäß Anspruch 1, bei der es sich um O⁶-(2-Benzo[b]thienylmethyl)guanosin handelt.

27. Verbindung gemäß Anspruch 1, bei der es sich um O⁶-(4-Picolylguanin handelt.

28. Verbindung gemäß Anspruch 1, bei der es sich um O⁶-(2-Naphthylmethyl)guanin handelt.

29. Pharmazeutische Zusammensetzung, die eine Verbindung gemäß einem der vorstehenden Ansprüche sowie eine pharmazeutisch annehmbare Trägersubstanz umfasst.

30. Pharmazeutische Zusammensetzung gemäß Anspruch 29, die weiterhin ein Alkylierungsmittel umfasst.

31. Verbindung gemäß einem der Ansprüche 1 bis 28 zur Verwendung bei einem Verfahren zur Abreicherung von O⁶-Alkylguanin-DNA-Alkyltransferase-Aktivität in einem Wirt.

32. Verwendung einer Verbindung gemäß einem der Ansprüche 1 bis 28 bei der Herstellung eines Medikaments zur Abreicherung von O⁶-Alkylguanin-DNA-Alkyltransferase-Aktivität in Tumorzellen.

33. Verwendung einer Verbindung gemäß einem der Ansprüche 1 bis 28 und eines Alkylierungsmittels bei der Herstellung einer ein- oder zweikomponentigen pharmazeutischen Zusammensetzung zur Behandlung von Tumorzellen.

34. Verwendung gemäß Anspruch 32 oder 33, wobei es sich bei der Verbindung gemäß einem der Ansprüche 1 bis 28 um O⁶-Thenylguanin oder ein substituiertes Derivat davon mit Substitution im Thiophenring durch eine Halogen-, Cyan- oder Estergruppe der Formel -COOR⁵, wobei R⁵ = C₁-C₂₀-Alkyl ist, handelt.

35. Zusammensetzung gemäß Anspruch 30, wobei das Alkylierungsmittel aus 1,3-Bis(2-chlorethyl)-1-nitrosoharnstoff (BCNU) und Temozolomid ausgewählt ist.

36. Zusammensetzung gemäß einem der Ansprüche 32 bis 34, wobei das Alkylierungsmittel aus 1,3-Bis(2-chlorethyl)-1-nitrosoharnstoff (BCNU) und Temozolomid ausgewählt ist.

37. Verfahren zur Herstellung einer Verbindung der Formel I gemäß Anspruch 1, umfassend das Umsetzen von Natriumhydrid mit einer Lösung von RR'CHOH (wobei R und R' wie in Anspruch 1 definiert sind) in einem organischen Lösungsmittel, das Hinzufügen von 2-Amino-N,N,N-trimethyl-1H-purin-6-aminiumchlorid oder 2-Amino-6-chlorpurinribosid, Behandlung mit schwacher Säure und Ether und Extrahieren des gewünschten Produkts.

## Revendications

1. Dérivés de O⁶-alkylguanine de formule I : 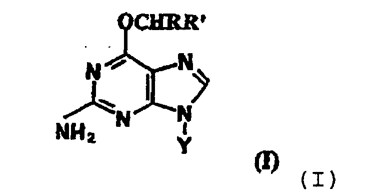 dans laquelle
Y représente H, le ribosyle, le désoxyribosyle ou R''XCHR''', dans laquelle X représente O ou S, R'' et R''' sont des groupes alkyle en C₁ à C₂₀ ou des dérivés substitués de celui-ci ayant été substitués par un ou plusieurs groupe(s) hydroxy, alkoxy en C₁ à C₂₀, amino, aminoalkyle en C₁ à C₂₀, amido ou uréido ;
R' représente H ou un groupe alkyle en C₁ à C₂₀ ou un groupe hydroxyalkyle en C₁ à C₂₀ ;
R représente :
(i) un groupe cyclique ayant au moins un cycle hétérocyclique composé de 5 ou 6 membres, de manière facultative avec un cycle carbocyclique ou hétérocyclique attaché à celui-ci ; le ou chaque cycle hétérocyclique ayant au moins un hétéroatome choisi parmi le groupe comprenant O, N ou S ou un dérivé substitué de celui-ci ayant été substitué dans le(s) cycle(s) hétérocyclique(s) et/ou cycle(s) carbocyclique(s) par un ou plusieurs groupe(s) alkyle en C₁ à C₂₀, alkényle en C₁ à C₂₀, alkynyle en C₁ à C₂₀, halo, haloalkyle en C₁ à C₂₀, nitro, cyano, hydroxyalkyle en C₁ à C₂₀, SOₙR'''' où R'''' est un groupe alkyle en C₁ à C₂₀ et n vaut 0, 1 ou 2 ou un groupe carboxyle ou ester de formule -COOR⁵ dans laquelle R⁵ représente H ou un groupe alkyle en C₁ à C₂₀ ; ou
(ii) un naphtyle ou un dérivé substitué de celui-ci ayant été substitué par un ou plusieurs groupe(s) alkyle en C₁ à C₂₀, alkényle en C₁ à C₂₀, alkynyle en C₁ à C₂₀, halo, haloalkyle en C₁ à C₂₀, nitro, cyano, hydroxyalkyle en C₁ à C₂₀, SOₙR'''' où R'''' est un groupe alkyle en C₁ à C₂₀ et n vaut 0, 1 ou 2 ou un groupe carboxyle ou ester de formule -COOR⁵ dans laquelle R⁵ représente H ou un groupe alkyle en C₁ à C₂₀ ;
et des sels pharmaceutiquement acceptables de ceux-ci.

2. Composé selon la revendication 1 , dans lequel :
Y représente H, le ribosyle, le désoxyribosyle ou R''XCHR''', dans laquelle X représente O ou S, R'' et R''' sont des groupes alkyle(s) en C₁ à C₂₀ ou des dérivés substitués de celui-ci ayant été substitués par un ou plusieurs groupe(s) hydroxy, amino, aminoalkyle en C₁ à C₂₀, amido ou uréido ;
R' est comme défini dans la revendication 1 ;
R représente :
(i) un groupe cyclique ayant au moins un cycle hétérocyclique composé de 5 ou 6 membres, de manière facultative avec un cycle carbocyclique ou hétérocyclique attaché à celui-ci ; le ou chaque cycle hétérocyclique ayant au moins un hétéroatome choisi parmi le groupe comprenant O, N ou S ou un dérivé substitué de celui-ci ayant été substitué dans le(s) cycle(s) hétérocyclique(s) et/ou cycle(s) carbocyclique(s) par un ou plusieurs groupe(s) alkyle en C₁ à C₂₀, alkényle en C₁ à C₂₀, alkynyle en C₁ à C₂₀, halo, haloalkyle en C₁ à C₂₀, nitro, cyano, hydroxyalkyle en C₁ à C₂₀, SOₙR'''' où R'''' est un groupe alkyle en C₁ à C₂₀ et n vaut 0, 1 ou 2 ; ou
(ii) un naphtyle ou un dérivé substitué de celui-ci ayant été substitué par un ou plusieurs groupe(s) alkyle en C₁ à C₂₀, alkényle en C₁ à C₂₀, alkynyle en C₁ à C₂₀, halo, haloalkyle en C₁ à C₂₀, nitro, cyano, hydroxyalkyle en C₁ à C₂₀, SOₙR'''' où R'''' est un groupe alkyle en C₁ à C₂₀ et n vaut 0, 1 ou 2 ;
et des sels pharmaceutiquement acceptables de ceux-ci.

3. Composé selon la revendication 1 ou 2 dans lequel R représente :
(a) un cycle hétérocyclique composé de 5 ou 6 membres ou
(b) un dérivé benzo de celui-ci, le groupe O⁶-alkylguanine étant attaché à R soit au niveau de l'hétérocycle, soit au niveau du cycle benzénique.

4. Composé selon la revendication 1 ou 2 dans lequel R représente un cycle hétérocyclique composé de 5 membres, contenant au moins un atome S ou un dérivé substitué de celui-ci selon la revendication 1 ou 2 respectivement.

5. Composé selon la revendication 1 ou 2 dans lequel R est un cycle thiophène ou un dérivé substitué de celui-ci selon la revendication 1 ou 2 respectivement.

6. Composé selon la revendication 5 dans lequel R représente un cycle thiophène substitué dans le cycle par un groupe halo.

7. Composé selon la revendication 6 dans lequel le groupe halo est iodo, bromo, chloro ou fluoro.

8. Composé selon la revendication 1 ou 2 dans lequel R est un cycle furane ou un dérivé substitué de celui-ci selon la revendication 1 ou 2 respectivement.

9. Composé selon la revendication 1 ou 2 dans lequel R'''' comprend un cycle hétérocyclique et/ou carbocyclique substitué par un groupe halo, un groupe haloalkyle en C₁ à C₂₀, un groupe cyano, SOₙR'''' dans lequel R représente un groupe alkyle en C₁ à C₂₀ et n vaut 0, 1 ou 2 ou -COOR⁵ dans laquelle R⁵ représente un groupe alkyle en C₁ à C₂₀.

10. Composé selon la revendication 1 ou 2 dans lequel R comprend un cycle hétérocyclique substitué par un groupe halo, cyano ou un groupe ester de formule -COOR⁵ dans laquelle R⁵ est un groupe alkyle en C₁ à C₂₀.

11. Composé selon l'une quelconque des revendications précédentes dans lequel R' représente H.

12. Composé selon la revendication 1 ou 2 qui est O⁶-thénylguanine ou un dérivé substitué de celui-ci substitué dans le cycle thiophène par un groupe halo, cyano ou un groupe ester.

13. Composé selon la revendication 12 qui est O⁶-thénylguanine.

14. Composé selon la revendication 12 qui est O⁶-thénylguanine ayant été substitué dans le cycle thiophène par un groupe halo.

15. Composé selon la revendication 14 dans lequel le groupe halo est iodo, bromo, chloro ou fluoro.

16. Composé selon la revendication 1 qui est O⁶-(3-thiénylméthyl)guanine.

17. Composé selon la revendication 1 qui est O⁶-pipéronyiguanine.

18. Composé selon la revendication 1 qui est O⁶-furfurylguanine.

19. Composé selon la revendication 1 qui est O⁶-(3-furylméthyl)guanine.

20. Composé selon la revendication 1 qui est O⁶-(2-benzo[b]thiénylméthyl)guanine.

21. Composé selon la revendication 1 qui est O⁶-(2-benzofuranylméthyl)guanine.

22. Composé selon la revendication 1 qui est O⁶-(5-thiazolylméthyl)guanine.

23. Composé selon la revendication 1 qui est O⁶-(5-méthoxycarbonylfurfuryl)guanine.

24. Composé selon la revendication 1 qui est O⁶-(5-bromothényl)guanine.

25. Composé selon la revendication 1 qui est O⁶-(5-cyanofurfuryl)guanine.

26. Composé selon la revendication 1 qui est O⁶-(2-benzo[b]thiénylméthyl)guanosine.

27. Composé selon la revendication 1 qui est O⁶-(4-picolyl)guanine.

28. Composé selon la revendication 1 qui est O⁶-(2-naphtylméthyl)guanine.

29. Composition pharmaceutique comprenant un composé selon l'une quelconque des revendications précédentes et un excipient pharmaceutiquement acceptable.

30. Composition pharmaceutique selon la revendication 29 comprenant en outre un agent alkylant.

31. Composé selon l'une quelconque des revendications 1 à 28 pour une utilisation dans un procédé de réduction de l'activité de O⁶-alkylguanine-ADN alkyltransférase chez un hôte.

32. Utilisation d'un composé selon l'une quelconque des revendications 1 à 28, dans la fabrication d'un médicament pour la réduction de l'activité de O⁶-alkylguanine-ADN alkyltransférase dans des cellules tumorales.

33. Utilisation d'un composé selon l'une quelconque des revendications 1 à 28 et d'un agent alkylant dans la fabrication d'une composition pharmaceutique à une ou deux parties pour le traitement de cellules tumorales.

34. Utilisation selon la revendication 32 ou 33 dans laquelle le composé selon l'une quelconque des revendications 1 à 28 est O⁶-thénylguanine ou un dérivé substitué de celui-ci ayant été substitué dans le cycle thiophène par un groupe halo, cyano ou un groupe ester de formule -COOR⁵ dans laquelle R⁵ représente un groupe alkyle en C₁ à C₂₀.

35. Composition selon la revendication 30 dans laquelle l'agent alkylant est choisi parmi le groupe comprenant la 1,3 bis(2-chloroéthyl)-1-nitroso-urée (BCNU) et la témozolomide.

36. Utilisation selon l'une quelconque des revendications 32 à 34 dans laquelle l'agent alkylant est choisi parmi le groupe comprenant 1,3 bis(2-chloroéthyl)-1-nitroso-urée (BCNU) et la témozolomide.

37. Procédé de préparation d'un composé de formule I selon la revendication 1 qui comprend la réaction entre l'hydrure de sodium et une solution de RR'CHOH (dans laquelle R et R' sont comme définis dans la revendication 1) dans un solvant organique, en ajoutant du chlorure de 2-amino-N,N,N-triméthyl-1H-purin-6-aminium ou du 2-amino-6-chloropurine riboside, le traitement avec un acide faible et de l'éther et l'extraction du produit désiré.
